# EUROPEAN PATENT SPECIFICATION

(11) **EP 3 325 643 B1**
(45) Date of publication and mention of the grant of the patent: **09.09.2020**
(21) Application number: 16738211.8
(22) Date of filing: 01.07.2016
(51) Int. Cl.: C12Q 1/6886

(54) **METHODS AND MEANS FOR DYSPLASIA ANALYSIS**
VERFAHREN UND MITTEL ZUR DYSPLASIEANALYSE
PROCÉDÉ ET MOYENS D'ANALYSE DE DYSPLASIE

(30) Priority: 24.07.2015 GB 201513060
(43) Date of publication of application: 30.05.2018
(73) Proprietor: United Kingdom Research and Innovation, Swindon SN2 1FL (GB)
(72) Inventor: FITZGERALD, Rebecca C., Cambridge Cambridgeshire CB2 2XZ (GB); VARGHESE, Sibu, Cambridge Cambridgeshire CB2 0 XZ (GB); NEWTON, Richard, Cambridge Cambridgeshire CB2 0 XZ (GB); WERNISCH, Lorenz, Cambridge Cambridgeshire CB2 0 XZ (GB)
(74) Representative: Script IP Limited
(86) International application number: PCT/GB2016/052007
(87) International publication number: WO 2017/017404

(56) References cited:
- HUMMERT C ET AL: "Creation and Comparison of Different Chip Definition Files for Affymetrix Microarrays", 2011 INTERNATIONAL CONFERENCE ON BIOINFORMATICS & COMPUTATIONAL BIOLOGY. BIOCOMP 2011, 18-21 JULY 2011, LAS VEGAS NV, USA,, vol. 1, 1 January 2011 (2011-01-01), pages 16-22, XP009186218, ISBN: 1-60132-170-8
- SU HUA ET AL: "Global gene expression profiling and validation in esophageal squamous cell carcinoma and its association with clinical phenotypes.", CLINICAL CANCER RESEARCH : AN OFFICIAL JOURNAL OF THE AMERICAN ASSOCIATION FOR CANCER RESEARCH 1 MAY 2011, vol. 17, no. 9, 1 May 2011 (2011-05-01), pages 2955-2966, XP002762156, ISSN: 1078-0432
- KASHYAP M K ET AL: "Genomewide mRNA profiling of esophageal squamous cell carcinoma for identification of cancer biomarkers", CANCER BIOLOGY AND THERAPY 2009 LANDES BIOSCIENCE USA, vol. 8, no. 1, 2009, pages 34-46, XP55304437, ISSN: 1538-4047
- VARGHESE S ET AL: "DEFINING CANCER RISK IN BARRETT'S OESOPHAGUS USING A 90-GENE SIGNATURE", GUT, vol. 62, no. Suppl. 1, June 2013 (2013-06) , page A12, XP002762154, & ANNUAL GENERAL MEETING OF THE BRITISH-SOCIETY-OF-GASTROENTEROLOGY; GLASGOW, UK; JUNE 24 -27, 2013
- REDMAN JAMES E ET AL: "Biomarkers for Early Detection of Esophageal Squamous Cell Dysplasia and Carcinoma", GASTROENTEROLOGY, vol. 146, no. 5, Suppl. 1, May 2014 (2014-05), page S96, XP002762155, & 55TH ANNUAL MEETING OF THE SOCIETY-FOR-SURGERY-OF-THE-ALIMENTARY-TRAC T (SSAT) / DIGESTIVE DISEASE WE; CHICAGO, IL, USA; MAY 03 -06, 2014
- HONG BIN WANG ET AL: "Research on the Typical miRNA and Target Genes in Squamous Cell Carcinoma and Adenocarcinoma of Esophagus Cancer with DNA Microarray", PATHOLOGY ONCOLOGY RESEARCH, vol. 20, no. 2, 12 February 2014 (2014-02-12), pages 245-252, XP055304341, HU ISSN: 1219-4956, DOI: 10.1007/s12253-013-9688-z
- OKAJIMA WATARU ET AL: "Plasma microRNA profiles: Identification of miR-25 as a novel diagnostic and monitoring biomarker in esophageal squamous cell carcinoma", JOURNAL OF THE AMERICAN COLLEGE OF SURGEONS, vol. 219, no. 4, 17 July 2014 (2014-07-17) , XP029085292, ISSN: 1072-7515, DOI: 10.1016/J.JAMCOLLSURG.2014.07.635
- VARGHESE SIBU ET AL: "Analysis of Dysplasia in Patients With Barrett's Esophagus Based on Expression Pattern of 90 Genes", GASTROENTEROLOGY, vol. 149, no. 6, November 2015 (2015-11), page 1511, XP029308240, ISSN: 0016-5085, DOI: 10.1053/J.GASTRO.2015.07.053

## Description

### FIELD OF THE INVENTION

The invention is in the field of diagnosing or aiding the diagnosis of dysplasia, in particular oesophageal dysplasia, in a subject.

### BACKGROUND TO THE INVENTION

It is a problem to separate the different stages of dysplasia. The later stages of dysplasia (which precede adenocarcinoma) require clinical intervention. However, typically, earlier stages of dysplasia such as low-grade dysplasia (LGD) do not require clinical intervention but are instead referred for monitoring.

It is a problem in the art that pathologists often do not agree on the classification of LGD. It is a problem that a pathologist may refer a patient for monitoring too often. This involves potentially unnecessary endoscopy which is unpleasant and invasive for the patient, and costly to the health care provider. In other words, there is a problem of "over-diagnosis" in the art. This problem is illustrated by a recent Dutch study (Curvers 2010 Am J Gastroenterol 105:1523-30). The authors re-examined all LGDs which were classified by a first round of histopathology. Under rigorous examination, 85% of those LGDs were re-classified as "no risk". This illustrates the problem of over-diagnosis which is present in the art.

The problem of this burden on health services such the UK's National Health Service (NHS) is worsening, since new guidance for treatment urges practitioners to consider treating LGD. This is even more expensive and potentially more burdensome on the health service than the previous guidance which was to refer patients for endoscopic monitoring. In addition, treatment is not itself risk-free, so that receiving unnecessary treatment is itself a problem in the art.

Approximately 0.44-0.49 % per year of patients with NDBE develop oesophageal adenocarcinoma. By contrast, approximately 13.4% per year of patients with low grade dysplasia (LGD) develop oesophageal adenocarcinoma.

Current clinical practice is to place patients having Barrett's Oesophagus under surveillance. These subjects are periodically examined. If no dysplasia is observed, they are returned to the surveillance programme. If high grade dysplasia is observed, the subject is referred for treatment such as radio frequency ablation of the high grade dysplasia lesion. Most importantly, if a subject is considered to have low grade dysplasia the current (and only) clinical way forward is to have multiple histopathologists examine samples from the same subject and to try to reach consensus. "Consensus LGD" is reached in only about 15% of cases. Firstly, this is an extremely laborious and resource intensive process since histopathologists are highly trained and experienced individuals and each analysis they perform is time consuming and therefore costly. Secondly, this demonstrates extreme variability in the state of the art system since a consequence of only 15% of cases being confirmed to contain LGD when reviewed by two expert gastrointestinal pathologists is that a remarkable 85% of patients are wrongly diagnosed (over diagnosed) by the initial assessment. This is clearly unsatisfactory in terms of a burden on the health service. This is also problematic in terms of the waste of resources in reclassifying the patients by histopathological consensus. There is also the drawback of increased trauma on the subjects involved being initially told they have low grade dysplasia, only for 85% of those subjects to be later told that in fact they did not.

Thus, there are currently two paths to treatment. The first path is by a positive diagnosis of high grade dysplasia. The second path is by the laborious and time consuming process of establishing "consensus LGD". It is a problem in the art to reliably identify LGD.

Varghese's et al scientific publication ( Gut, & Annual General Meeting Of The British-Society-Of-Gastroenterology; Glasgow, UK; June 24 -27, 2013, vol. 62, no. Suppl. 1, page A12) discloses defining cancer risk in Barrett's oesophagus using a 90-gene signature.

Redma's et al scientific publication Gastroenterology, & 55th Annual Meeting Of The Society-For-Surgery-Of-The-Alimentary-Tract (Ssat) / Digestive Disease We; Chicago, IL, USA; May 03 -06, 2014, vol. 146, no. 5, Suppl. 1, page S96) discloses biomarkers for early detection of esophageal squamous cell dysplasia and carcinoma.

Su Hua's scientific publication ( Clinical Cancer Research : An Official Journal Of The American Association For Cancer Research 1 May 2011, vol. 17, no. 9, ISSN 1078 -0432, pages 2955 - 2966) discloses global gene expression profiling and validation in esophageal squamous cell carcinoma and its association with clinical phenotypes.

Kashyap's et al scientific publication (Cancer Biology And Therapy 2009 Landes Bioscience USA, (2009), vol. 8, no. 1, ISSN 1538-4047, pages 34 - 46) discloses genomewide mRNA profiling of esophageal squamous cell carcinoma for identification of cancer biomarkers.

Hummert's et al scientific publication (International Conference on Bioinformatics & Computational Biology. Biocomp 2011, 18-21 July 2011, Las Vegas NV, USA, vol. 1, ISBN 1-60132-170-8, pages 16 - 22) discloses creation and comparison of different chip definition files for Affymetrix microarrays.

### SUMMARY OF THE INVENTION

In contrast, the present inventors realised that there are difficulties with the current classification of lesions in Barrett's esophagus ranging from LGD to HGD and on to adenocarcinoma. The inventors realised that there are problems and disagreements between histopathologists in classifying LGD and other early stage lesions.

The inventors studied this problem in considerable depth. They went on to use a strict consensus scoring (multiple histopathological analysis) system in order to rigorously identify NDBE and higher risk dysplasia. As a result of their studies, the inventors have designed a new system which aims to simply categorise patients as low risk or high risk. This system has the advantage of overcoming problems with disagreement of classification between LGD, HGD and other dysplasias. Instead, the inventors have taken a completely different view and have reformulated the problem as to which "LGD" patients are at high risk and which are at low risk.

In order to address this new problem, the inventors have designed a new gene signature analysis which is able to separate patients from a low risk category (corresponding to non-dysplastic Barrett's Oesophagus (NDBE)) from those having a high risk (i.e. having high grade dysplasia/HGD). This new approach was borne from the inventors' insight that the range of conditions from NDBE to adenocarcinoma is actually a continuum which defies robust classification along conventional LGD lines. Instead, the inventors seek to identify the high risk patients and the low risk patients and to separate them using a simple single test.

Thus the present invention provides a direct test for aiding identification of those subjects having dysplasia, such as dysplasia in need of intervention. The present invention provides a specific and defined set of genes which together form a signature which diagnoses, or aids in the diagnosis of, dysplasia.

Thus in one aspect the invention relates to a method of aiding the diagnosis of dysplasia in a subject, said method comprising:
(a) providing an in vitro sample from the oesophagus of said subject;
(b) assaying said sample for expression of each of the genes shown in the '40 genes' column of Table 1;
(c) normalising the expression levels of the genes in part (b) to expression levels of reference gene(s) from a non-dysplastic sample;
(d) determining from the normalised expression levels of (c) a gene signature score for the sample, wherein a gene signature score greater than a reference threshold indicates presence of dysplasia in said subject.

Suitably the gene signature score is determined by taking a weighted average of the 40 normalized ΔCt values. Suitably the weights are the normalized t-statistics from the training data Limma analysis; weights range in absolute value from 1 to 0.501 (see table 2).

Suitably the reference threshold is determined via the Youden's statistic or the 'closest-top left'; most suitably via the Youden's statistic.

Suitably said sample is a biopsy.

Suitably said biopsy is a pinch biopsy, or an endoscopic brushing.

In one embodiment suitably providing a sample from the oesophagus of said subject comprises using a biopsy collection device such as a pinch biopsy collector, introducing said device into the oesophagus of the subject, collecting the biopsy such as pinch biopsy, and retrieving same from the subject.

In one embodiment the sample is an *in vitro* sample previously collected and in this embodiment suitably the invention does not involve interaction with the subject's body.

Suitably assaying for expression of said genes is carried out by quantification of nucleic acid such as RNA in said sample.

Suitably assaying for expression of said genes is carried out using Fluidigm™ analysis.

Suitably said assay includes Gaussian normalisation, suitably step (c) comprises Gaussian normalisation.

Suitably assaying for expression of said genes is carried out using an expression array.

Suitably assaying for expression of said genes is carried out using RNA sequencing such as RNASeq.

Suitably the expression of the genes is assayed by detection of the probe(s) for said genes as shown in Table 2 and/or wherein the expression of the genes is assayed using the TaqMan™ Assay IDs as shown in Table 2.

Suitably said sample comprises RNA extracted from cell(s) of said subject.

Suitably said subject has Barrett's Oesophagus.

Also described is a set of nucleic acid probe(s) capable of detecting nucleic acid such as RNA from each of the genes shown in the '40 genes' column of Table 1.

Also described is a composition or set of compositions comprising at least one nucleic acid primer for the amplification or sequencing of each of the genes shown in the '40 genes'column of Table 1.

In one aspect, the invention relates to an array consisting of nucleic acid probe(s) capable of detecting RNA from each of the genes shown in the '40 genes' column of Table 1, wherein said array comprises a biochip to which the nucleic acid probe(s) are immobilised.

Also described is a device comprising a set of nucleic acid probes as described above, or a composition or set of compositions as described above, or an array as described above.

In one aspect, the invention relates to a method as described above wherein step (b) comprises contacting nucleic acid of said sample with one or more isolated probe(s) to allow hybridisation/binding to said probe(s), and then reading out said binding/hybridisation.

In one aspect, the invention relates to a method of aiding identification of a subject at risk of developing oesophageal adenocarcinoma, said method comprising performing the method as described above wherein presence of dysplasia in said subject indicates that said subject is at risk of developing oesophageal adenocarcinoma.

In one aspect, the invention relates to a computer program product operable, when executed on a computer, to perform the method steps (c) to (d) as described above. In one aspect, the invention relates to a data carrier or storage medium carrying a computer program product as described above.

### FURTHER ASPECTS

The methods of the invention may be applied to patients suspected of having low grade dysplasia (LGD). Thus, in this aspect the methods of the invention may directly replace the "consensus LGD" process which is currently the only way of clinically establishing LGD in a subject in need of treatment.

The invention maybe applied to a direct surveillance (population surveillance), for example the methods of the invention may be directly applied to subjects having Barrett's Oesophagus. This provides the advantage of eliminating a histopathological step of examining cells from a patient for morphology to try to classify them into no dysplasia/LGD (with all the attendant problems as explained above)/HGD. In this way, the methods of the invention may be directly performed on subjects (e.g. performed on samples such as *in vitro* samples obtained from, or provided from, subjects) having Barrett's Oesophagus thereby simplifying the transfer of patients between BE surveillance and treatment for dysplasia.

Also described is a method of aiding the diagnosis of dysplasia in a subject, said method comprising:
(a) providing a sample from the oesophagus of said subject;
(b) assaying said sample for expression of each of the genes shown in the '40 genes' column of Table 1;
(c) normalising the expression levels of the genes in part (b) to expression levels of reference gene(s) from a non-dysplastic sample;
(d) determining from the normalised expression levels of (c) a gene signature score for the sample, wherein a gene signature score greater than a reference threshold indicates high risk of presence of dysplasia in said subject.

Also described is a method of aiding the diagnosis of dysplasia in a subject, said method comprising:
(a) providing a sample from the oesophagus of said subject;
(b) assaying said sample for expression of each of the genes shown in the '40 genes' column of Table 1;
(c) normalising the expression levels of the genes in part (b) to expression levels of reference gene(s) from a non-dysplastic sample;
(d) determining from the normalised expression levels of (c) a gene signature score for the sample, wherein a gene signature score lower than a reference threshold indicates low risk of presence of dysplasia in said subject.

The invention may be a method of diagnosing, or may be a method of aiding the diagnosis of, dysplasia in a subject.

Suitably the dysplasia is oesophageal dysplasia.

Suitably the dysplasia is surface oesophageal dysplasia.

Suitably the dysplasia is oesophageal epithelial dysplasia.

### DETAILED DESCRIPTION OF THE INVENTION

The invention relates to a 40-gene signature which provides an objective adjunct to histopathology to diagnose dysplasia in Barrett's Esophagus; a 40-gene signature to diagnose dysplasia in Barrett's esophagus.

Advantageously, the number of genes in the signature has been optimized for considerations of convenient chip size for analysis.

Suitably, the number of genes in the signature has been optimized according to statistical criteria explained below.

The invention relates to diagnosis (or aiding the diagnosis) of dysplasia in a subject.

The methods of the invention provide diagnosis (or aiding of diagnosis) of dysplasia in a subject.

Suitably the subject has Barrett's Oesophagus.

Suitably the subject is in a Barrett's Oesophagus surveillance programme.

The methods are useful to identify the "true low grade group". These are subjects who progress to cancer if they do not receive treatment. These are subjects who harbour dysplasia.

A positive finding in the methods described herein indicates progression towards EAC.

A positive finding in the methods described herein indicates presence of dysplasia.

A positive finding in the methods described herein indicates that treatment should be administered to the subject. Treatment may be for example radio frequency ablation of the lesions such as the dysplastic area.

At the date of filing of this application, the only known method for identification of dysplasia such as LGD is histopathological consensus. Apart from the positive identification of high grade dysplasia, this is currently the only way of identifying patients in need of treatment. The methods of the present invention provide an advantageous new and reliable method for identifying patients in need of treatment.

Analysis of K values which are used to assess agreement between pathologists clearly identifies a problem which is addressed by the present invention i.e. a frequent lack of agreement between pathologists regarding patient classification.

### BARRET'S ESOPHAGUS

Barrett's esophagus (BE) has a highly variable outcome with 0.12-0.5% of patients per year progressing to esophageal adenocarcinoma (EA). The long term survival of patients diagnosed with symptomatic EA remains poor. The purpose of endoscopic surveillance in patients with BE is to identify those at risk of progressing to cancer at an early, curable stage. Currently this relies on the histopathological diagnosis of dysplasia. The grading of dysplasia is based on the Vienna classification which takes into account a number of cytological and tissue architectural features in the sample. The assessment of these features can be subjective and hence contribute to considerable intra- and inter-observer variability in the reporting of dysplasia. Low grade dysplasia (LGD) has been shown to be commonly over diagnosed by general pathologists with high levels of variability between pathologists. Curvers *et al.* demonstrated that only 15% of BE cases diagnosed with LGD were confirmed to contain LGD when reviewed by two expert gastrointestinal pathologists, suggesting that 85% of patients were over-diagnosed. Importantly, the incidence of high grade dysplasia (HGD) or cancer was 13.4% per patient per year in those in whom the diagnosis of LGD was confirmed compared to 0.49% per patient per year in those who following a consensus review were down-graded to non-dysplastic Barrett's esophagus (NDBE)⁶. Another study reported a cancer incidence rate of 0.44% per year in those diagnosed with LGD. In this case, however, expert pathology review did not influence patient outcome, although the κ value among pathologists for the diagnosis of LGD in this study was worryingly low at 0.14 confirming the difficulty in assigning this diagnosis. Several other studies spanning more than 20 years have highlighted the inter-observer variability in the diagnosis of dysplasia in BE. Given that LGD is currently the only accepted predictor for neoplastic progression prior to the point of intervention, it is crucial to identify this group of "true LGD" patients in a more definitive manner. The interim results from a randomized control trial suggest that there is a significantly reduced risk of neoplastic progression in stringently confirmed LGD cases that were treated with radiofrequency ablation. Hence, if this high-risk group can be identified with more certainty then there would likely be a case for more widespread acceptance for treatment of patients at this early stage with ablative therapy. As dysplasia is the cellular manifestation of multiple underlying genetic changes, the inventors reasons that a more direct measure of molecular factors might logically be a better indicator of cancer risk. Depending on the assay, a molecular test would also have the potential to provide a more objective risk stratification than the current histological assessment of dysplasia. In various pathological contexts the expression patterns of genes from microarray data have been shown to be powerful tools as biomarkers using the class prediction model. The class prediction model refers to formulating a rule with a set of genes often called a 'gene-signature' or 'classifier' that can distinguish different classes of disease. A combination of levels or weights applied to the genes yield a score. If a score is above a certain threshold the specimen would be classified into one category and if the score is below the threshold it would fall into the other category.

Such gene signatures have been shown to be useful in classifying different types of tumors, predicting response to chemotherapy and outcome. The breast cancer gene-expression signature is an example whereby a microarray-based signature proved to be a more powerful predictor of disease outcome than other clinical parameters. Another example is in the characterization of thyroid nodules. A prospective, multicenter study showed that a microarray based gene-expression signature was a powerful tool in classifying thyroid nodules with indeterminate cytology on fine-needle aspiration. Of the 265 indeterminate nodules, 85 were ultimately proven to be malignant and the gene-signature correctly identified 78 of them correctly [92% sensitivity (95% CI, 84-97); 52% specificity (95% CI, 44-59)]. The management of thyroid nodules with indeterminate cytology poses a dilemma to the clinician. The use of this gene-signature would appropriately favour the conservative approach in a majority of patients. This is analogous to the situation with the present invention in which we identify a more objective biomarker for LGD which is notoriously difficult to grade accurately, with the advantage that this approach is industrially applicable as an adjunct to histopathology and thereby inform decision making with regards to the optimal surveillance intervals and the suitability of a patient for ablative therapy.

### SAMPLE

Suitably the sample is a biopsy provided from the subject.

Suitably the method may involve collection of the biopsy sample.

More suitably, the method does not involve direct collection of the biopsy but is performed on an *in vitro* sample provided from the subject of interest.

Suitably the biopsy may be a pinch biopsy.

Suitably the biopsy may be an endoscopic brushing.

Suitably the biopsy material is frozen.

Suitably the sample comprises RNA from the subject.

RNA preparation is well known in the art.

Suitably the sample is from the oesophagus.

Suitably the sample is from the region of the oesophagus suspected of harbouring possible dysplasia.

RNA can be challenging to extract from paraffin embedded samples. If the sample is paraffin embedded, special care must be taken in RNA preparation.

It is known to collect material from the oesophageal lumen using a compressible abrasive material, such as a "cytosponge" or similar device. For example, see WO2011/058316 which discloses a cytosponge having particular hitch knot attachment means or WO2007/045896 discloses a cytosponge more generally. If the sample is collected using such a device, then appropriate techniques should be used to extract the RNA from the cells so collected. RNA preparation is well known in the art.

Suitably the sample is, or is derived from, endoscopic brushings frozen after collection.

Most suitably the sample is, or is derived from, a pinch biopsy frozen after collection.

Suitably the sample comprises RNA such as purified RNA or extracted RNA.

Suitably the sample consists essentially of RNA such as purified RNA or extracted RNA. Suitably the sample consists of RNA such as purified RNA or extracted RNA.

Suitably RNA extraction may be carried out using PicoPure™ RNA isolation kit from Applied Biosystems™ according to the manufacturer's instructions.

Suitably RNA extraction may be carried out using miRNeasy™ Mini Kit RNA isolation kit from Qiagen™ according to the manufacturer's instructions.

### ASSAY PLATFORM

In principle any platform capable of measuring nucleic acids such as RNA abundance may be used in the assays described. In particular any suitable technique for assessing RNA abundance may be used for assaying said sample for expression of the gene(s) of interest. For example, arrays such as micro-arrays may be used. For example, nucleic acid chips such as custom nucleic acid chips may be used. For example, RNA sequencing such as "RNA-seq" may be used. RNA-seq (RNA Sequencing), also called Whole Transcriptome Shotgun Sequencing (WTSS), is a technology that uses the capabilities of next-generation sequencing to reveal a snapshot of RNA presence and quantity from a genome at a given moment in time, and techniques for carrying out this analysis is well known in the art.

Suitably the analytical platform is capable of providing quantitative information regarding the RNA transcripts in the sample.

Fluidigm™ is an especially useful platform for carrying out assays as described herein. Suitably the Fluidigm™ qPCR array is used, for example as supplied by Fluidigm™, 7000 Shoreline Court, Suite 100, South San Francisco, CA 94080, USA.

When an array platform is used in the assay of the invention, suitably said array may be an Agilent expression array, for example as supplied by Agilent Technologies, Inc., 5301 Stevens Creek Blvd, Santa Clara, CA 95051, United States.

When conducting the analysis using fluid IGM platform analysis, it may be useful to normalise the data between chips/between reads. The person skilled in the art will be aware of the need for normalisation to ensure consistent results between chips/between reads. It is noted that Gaussian normalisation may be advantageous, especially when used in conjunction with Fluidigm obtained data. Median normalisation may be used. More suitably Gaussian normalisation is used. Gaussian normalisation is discussed in more detail in the examples section.

Detection or assay of expression is suitably accomplished via contacting nucleic acid of said sample with one or more isolated probe(s) to allow hybridisation/binding to said probe(s), and then reading out said binding/hybridisation. Said probe(s) is/are suitably not nature-identical. Suitably said probe(s) is/are artificial nucleic acid sequence(s). Suitably said probe(s) is/are *in vitro* manufactured. Suitably said probe(s) is/are not naturally occurring sequences. For example suitably said probe(s) is/are complementary to naturally occurring sequences. Suitably said probe(s) comprise a non-naturally occurring addition such as a dye, a label or other non-natural detection moiety. Suitably said probe(s) is/are immobilised. Suitably said probe(s) is/are bound to a device such as a gene chip or array such as a microarray. Suitably said probe(s) is/are *in vitro* probes.

In one aspect the invention relates to probe(s) such as nucleic acid probe(s) capable of detecting nucleic acid such as RNA from each of the genes shown in the '40 genes' column of Table 1. Suitably the invention relates to a set of probe(s) such as nucleic acid probe(s) capable of detecting nucleic acid such as RNA from each of the genes shown in the '40 genes' column of Table 1.

### DATA ANALYSIS

For statistical analysis as described herein, any suitable calculation technique or algorithm may be used, most suitably the R package which comprises a suite of statistical tools may be used.

For example, to measure differential expression, any suitable technique known in the art may be used. For example, a standard t-test or a moderated t-test or any other statistically suitable analysis method may be used. For example, for analysis/determination of differential expression, Limma, which performs a moderated t-test, may be used. This is a standard technique in microarray analysis for reducing False Positives. The R package and/or the Limma program (which may be used within R) are both available as open source software for bioinformatics e.g. from providers such as Bioconductor at the Fred Hutchinson Cancer Research Center, 1100 Fairview Ave. N., P.O. Box 19024, Seattle, WA 98109, USA (http://wvw.biocondur.org/).

Classifiers besides a simple average of values, such as a support vector machine (SVM), k-nearest-neighbours and diagonal linear discriminant analysis may be used if desired. Choice is a matter for the skilled operator working the invention. In case any guidance is required, it should be noted that the SVM can give slightly higher AUC but the difference is typically not large and with SVM's there is a greater risk of overfitting which should be borne in mind if SVM's are used in the analysis. In addition, a possible disadvantage of an SVM is that it may not be platform independent. A classifier using a score calculated from an average is transferable, and so suitably a classifier based on an average is used. Suitably SVM's are not used.

It will be noted that each gene has a sign which has to be included when averaging, since some genes in the signature are up-regulated in dysplasia such as HGD compared to NDBE whereas others are down-regulated in dysplasia such as HGD compared to NDBE. These signs are represented with each gene as '-1' or '1' (or alternatively a simple minus sign or no minus sign when giving the 'weight' for a particular gene); the minus sign in front of the weight (or a separate '-1') designates a minus sign and refers to a gene which is down-regulated in dysplasia such as HGD compared to NDBE and the **absence** of a minus sign in front of the weight (or a separate '1') designates a **plus** sign and refers to a gene which is **up-regulated** in dysplasia such as HGD compared to NDBE. In other words, the 'sign', plus or minus, refers to the difference in expression between NDBE and dysplasia. If the gene is over expressed in dysplasia the sign is positive (plus or '1') and when under expressed in dysplasia the sign is negative (minus or '-1').

### CONTROL PROBES

When assaying gene expression, it is good practice to use control probes to normalise expression values. Any suitable control probe may be used. For example, RN18S1, GAPDH or POLR2 may be used as control probes. Multiple control probes may be used. Values from multiple control probes may be averaged, e.g. using median average.

For example, when the assay is carried out using a Fluidigm™ platform The qPCR values from the Fluidigm™ platform are normalised using control probes. A suitable configuration may have three control probes RN18S1, GAPDH and POLR2. The median value of all three control probes may be used to normalise. The median Ct value for the three control probes is subtracted from the Ct values of each of the gene signature probes to give deltaCt values for each of the gene signature probes.

Details of exemplary control probes (reference genes) are as follows:

| **Control Probe** (reference gene) | **Accession Number** | **Notes** |
|---|---|---|
| RN18S1 | NR_003286 | |
| GAPDH | NM_001256799 | |
| POLR2 | NM_000937 | |

Alternatively, or in addition, normalisation using the Universal Human Reference RNA (UHRR) may be carried out.

Suitably normalising the expression levels of the genes of the signature to expression levels of reference gene(s) from a non-dysplastic sample is carried out by normalising to one or more of RN18S1, GAPDH, POLR2 or UHRR, more suitably to one or more of RN18S1, GAPDH, POLR2.

Suitably normalising is to two or more such reference genes, more suitably to three or more such reference genes, more suitably to four or more such reference genes.

Suitably assaying said sample for expression comprises or includes the analysis of calibration samples to ensure that intensity values between different runs or reads are comparable.

Suitably the platform used to assay the sample for expression comprises two or more control probes or calibration samples; suitably 3 or more; suitably 4 or more; suitably 5 or more; suitably 6 or more; suitably 7 or more; suitably 8 or more; suitably 9 or more; suitably 10 or more control probes or calibration samples.

It may be helpful to use effectively a distribution of control probes.

In any case, it will be noted that using three control probes is already very good practice. It is unlikely that more than 5 would be used.

### Normalising expression levels

Preferably three reference genes should be assessed for each sample (e.g. RPS18, POLR2A, GAPDH) to normalise for cDNA input.

For each sample the median of the three reference genes' Ct values is used to calculate the ΔCt values for the 40 target genes (i.e. Ct of target gene - median Ct of reference genes).

Suitably each sample's 40 ΔCt values are then Gaussian normalized. That is, if rᵢ is the rank of the *i*th probe on the array, its value is Gaussian transformed to xᵢ where Pr(X < xᵢ) = rᵢ/41, and xᵢ are assumed to be distributed according to a standard Gaussian. Each sample's gene signature score is calculated by taking a weighted average of the 40 normalized ΔCt values.

Suitably the weights are the normalized t-statistics from the training data Limma analysis; weights ranged in absolute value from 1 to 0.501 (table 2).

### Determining a reference threshold (gene signature score)

The section below explains how to decide on a threshold.

### Deciding on a threshold:

In deciding a threshold to use the skilled operator should consider misclassification costs and the balance of the class distributions. In this case misclassification costs are such that we want to get zero HGD misclassifications, with a minimum number of NDBE misclassifications.

There are two main ways of deciding on the optimal cut-off:
Firstly Youden's statistic takes the optimal cut-off as the threshold that maximizes the distance to the identity (diagonal) line, that is:
   *max(sensitivities* + *specificities)*
Secondly closest-topleft, were the optimal threshold is the point closest to the top-left part of the plot with perfect sensitivity or specificity, that is:
   *min((1* - *sensitivities)²* + *(1* - *specificities)²)*

Both these formulae can be modified to adjust the optimal threshold taking into account misclassification costs. That is if the cost of a False Positive is different to the cost of a False Negative. Here we do not want any False Negatives since we don't want to miss any dysplasia (HGDs). The optimal threshold is also dependent on the prevalence of the disease (in this case dysplasia (HGD)) in the target test population (in this case patients with NDBE). The prevalence in this case is only about 0.5%.

The formulae are modified to:
*max(sensitivities* + *r* × *specificities)* for Youden
*min((1* - *sensitivities)²* + *r* × (*1* - *specificities)²)* for closest-topleft
where *r = (1-prevalence)*/*(cost*×*prevalence)*

We set prevalence to 0.005 and plot the calculated optimal threshold and Sensitivity and Specificity for a range of cost values.

In the figures of Youden's statistics, red dots show the optimal threshold, green dots the Specificity and black dots the Sensitivity.

Results for a 90 gene signature are shown in the examples. For a 90 gene signature, a Sensitivity of 1 is obtained with a threshold of -0.125. The specificity at this threshold is 0.157 (Youden and closest-topleft give identical values).

For a 70 gene signature the optimal threshold for a Sensitivity of 1 is -0.163 and the Specificity is 0.1765.

For a 60 gene signature the optimal threshold for Sensitivity of 1 is -0.225 and the Specificity is then 0.157.

In order to get a Sensitivity of 1 the Specificity drops dramatically.

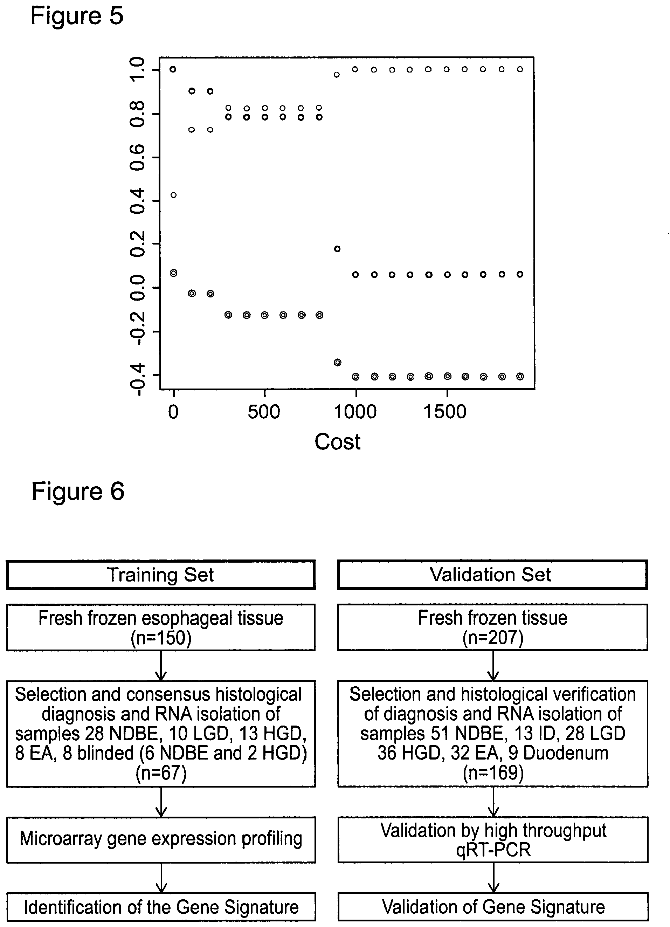

For a 40 gene signature see Figure 5. The red dots (all bottom dots) show the optimal threshold; green dots (first three upper dots, remainder middle dots) the Specificity; and black dots (first three middle dots, remainder upper dots) the Sensitivity.

In case any further guidance is required, for a 40 gene signature with high sensitivity the values can be taken as:
>> sensitivity = 1.000
>> specificity = 0.059
>> threshold (reference threshold)= -0.409

Alternatively, for a 40 gene signature for better specificity the values can be taken as:
>> sensitivity = 0.825
>> specificity = 0.784
>> threshold (reference threshold)= -0.124

### SEQUENCE INFORMATION

Unless otherwise apparent, accession numbers are for GenBank (GenBank, National Center for Biotechnology Information, National Library of Medicine, 38A, 8N805, 8600 Rockville Pike, Bethesda, MD 20894, USA. The database release is Genetic Sequence Data Bank, 15 June 2015; NCBI-GenBank Release Number 208.0

'Taqman™' assay IDs are well known in the art as used by Life Technologies (Thermo Fisher Scientific, Einsteinstrasse 55, Ulm 89077, Germany).

Celera annotations are well known in the art as used by Celera Alameda (1401 Harbor Bay Parkway, Alameda, CA, USA).

### CROSS-PLATFORM VALIDATION

It is an advantage of the method of the invention that it is equally applicable on different analysis platforms. This has been built in to the method by the inventors. For example, the training phase used in producing the gene signature was conducted on a first platform - a Taqman™ "all transcribed" gene set. This gene set typically comprises approximately 30,000 genes. The inventors then selected approximately 90 genes from this 30,000 gene pool. For the next phase of method design, the inventors decided to use an entirely fresh set of samples which had been totally independently collected from the training set. In addition, the inventors manufactured a custom chip on the fluid IGM platform. Thus, as part of the design of the method of the invention, entirely different subject samples were used in conjunction with an entirely different platform for their analysis, compared to the "training phase" aspect of the method design. This approach provides the technical benefit that the method is not tied to any particular platform and may be conveniently deployed across any suitable platform for analysis.

### CROSS VALIDATION

The inventors used different statistical classifiers on the training data. Indeed, they compared a number of different statistical classifiers applied to the same data set. The inventors were surprised to discover that they could obtain meaningful data using only a simple statistical classification algorithm. This was a surprise because the expectation would be that the more complex analysis would yield more reliable data. Using the simple algorithm to obtain reliable data was an unexpected benefit. This avoids the need for a more complicated analysis and provides the benefit that the method can be made more portable, i.e. the method can work across platforms which would not be the case if the expected more complex algorithm had been deployed. At a practical level, this solves the technical problem of having to re-train the signature on each different platform intended to be used for analysis.

### GENE SIGNATURE

The inventors advantageously identified a 40 gene signature. This signature has the technical benefit of reliably identifying dysplasia in subjects being examined. This also provides advantages in a more rapid and less labour intensive analysis, and provides advantages of cost savings and reduced use of consumables.

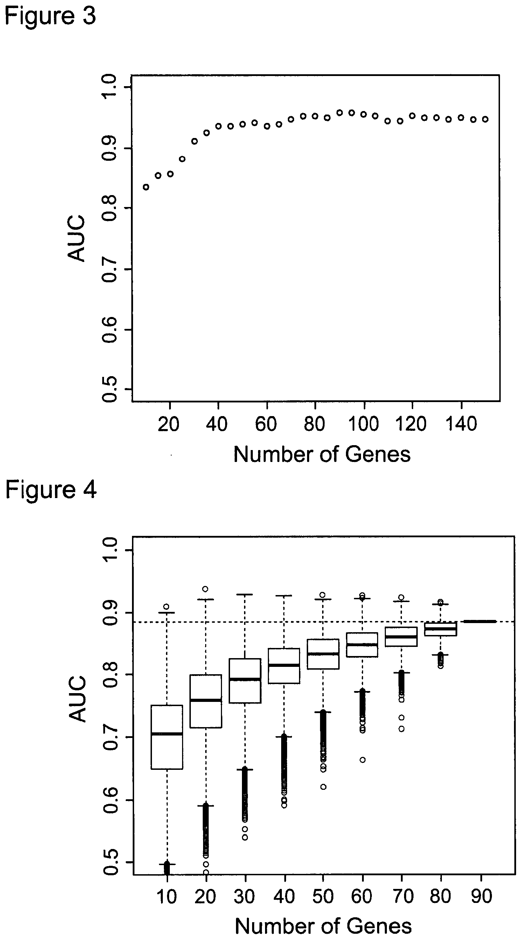

An advantage of the invention is the high value of the area under the curve (AUC), which measures the diagnostic accuracy of a panel, which is provided by the signature such as the 40 gene signature. As discussed in the examples section below, the AUC clearly starts to decrease below 40 genes. At 40 genes, the AUC is consistently above 90%. However, fewer genes causes a marked decrease, for example the AUC decreases below 90% for signatures below 35 genes. This is illustrated in Figure 3 and discussed in detail in Example 2 below. In addition, this list is specific for that combination of 40 genes.

AUC data presented herein show that a maximum AUC is reached at n=40 genes, and including more genes has only a modest or negligible effect on the classification ability of the signature. This further emphasises the unique qualities of the 40 gene signature.

The inventors further realised that occasionally technical issues can arise such as bubbles in a chip preventing a complete read of the chip. Therefore in some embodiments suitably an expanded gene set is assayed, for example a 60 gene, 63 gene 70 gene, 80 gene or 90 gene signature may be assayed. One example is the 90 gene signature disclosed (e.g. see '90 genes' column in table 1 below). This has the advantage of overcoming the further technical problem of incomplete reads. This has the further advantage of higher AUC with higher gene numbers towards 90.

Suitably the genes assayed are the following 40 (from the expression data):
TRUB2 CAST PARD6A RNF112 RAP2C DMXL1
MRPS23 SLC2A13 SF3B3 HCFC2 RGS2 PTPN2
ECT2 DDX28 QTRTD1 FPGS DAG1 NUP62
PTRH2 XPO5 C9orf3 TICAM2 SLC31A2 PRKDC
SIRT4 KIAA1191 SECTM1 HN1L PTGES2 APC
ZNF608 DDX27 ZSWIM6 COX7C STIP1 CCPG1
CSTF1 CDCA5 BRCA1 MAPK9

Suitably the genes assayed are the following 60 (from the expression data):
TRUB2 CAST PARD6A RNF112 RAP2C DMXL1
MRPS23 SLC2A13 SF3B3 HCFC2 RGS2 PTPN2
ECT2 DDX28 QTRTD1 FPGS DAG1 NUP62
PTRH2 XPO5 C9orf3 TICAM2 SLC31A2 PRKDC
SIRT4 KIAA1191 SECTM1 HN1L PTGES2 APC
ZNF608 DDX27 ZSWIM6 COX7C STIP1 CCPG1
CSTF1 CDCA5 BRCA1 MAPK9 FBXW11 SEC24A
FAM38A RG9MTD1 MCM2 SEC31A FAM63A HPGD
TMEM140 PPIP5K2 KPNA2 MYBL2 NOL11 XPO1
CITED2 TSN DCUN1D3 AKR1B10 CEP55 MKI67IP

Suitably the genes assayed are the following 63 (from the Fluidigm data):
SEC24A FPGS DDX28 RGS2 DTYMK HPGD
TSN CKS1A ZSWIM6 ECT2 MYBL2 BID
MKI67IP STARD4 DMXL1 TEP1 XPO5 CCPG1
FAM63A MCM2 KPNA2 HCFC2 APC PRPF4
STMN1 GDPD2 TMEM140 PTRH2 SLC2A13 FOXK2
RRM2 SERPINH1 AKR1B10 XPO1 CEP55 CDCA5
MAPK9 ASF1B SLC31A2 BRCA1 RCC2 CLK4
SAE1 NDUFA1 ZNF608 CSTF1 TRUB2 PTPN2
CAST KIAA1191 DDX27 SIRT4 SECTM1 COX7C
SDCCAG3 SF3B3 PPIP5K2 PTGES2 FAM38A FBXO45
C9orf3 CCDC43 POLE3

Suitably the genes assayed are the following 70:
TRUB2 CAST PARD6A RNF112 RAP2C DMXL1
MRPS23 SLC2A13 SF3B3 HCFC2 RGS2 PTPN2
ECT2 DDX28 QTRTD1 FPGS DAG1 NUP62
PTRH2 XPO5 C9orf3 TICAM2 SLC31A2 PRKDC
SIRT4 KIAA1191 SECTM1 HN1L PTGES2 APC
ZNF608 DDX27 ZSWIM6 COX7C STIP1 CCPG1
CSTF1 CDCA5 BRCA1 MAPK9 FBXW11 SEC24A
FAM38A RG9MTD1 MCM2 SEC31A FAM63A HPGD
TMEM140 PPIP5K2 KPNA2 MYBL2 NOL11 XPO1
CITED2 TSN DCUN1D3 AKR1B10 CEP55 MKI67IP
HEATR1 SAE1 CLK4 STMN1 DTYMK PRPF4
TBC1D9B FOXK2 PAQR4 POLE3

When using expanded gene groups such as 60, 63, 70 gene groups as exemplified above, the method(s) are exactly as exemplified for the preferred 40 gene group or 90 gene group - see examples section for further guidance. The methods exemplified should be modified only to accommodate alternate gene numbers, for example when normalising or performing other calculations the numbers used should be adapted accordingly to the number of genes examined which is well within the ambit of the skilled reader.

A list of gene designations is provided below in Table 1:

**Table 1:**

| **90 Genes** | **40 genes** | **35 genes (not part of invention)** | **7 genes (not part of invention)** |
|---|---|---|---|
| AKR1B10 | APC | APC | BID |
| APC | BRCA1 | BID | ECT2 |
| ASF1B | C9orf3 | CCPG1 | FBXW11 |
| BID | CAST | CKS1A | FPGS |
| BRCA1 | CCPG1 | DCUN1D3 | SLC31A2 |
| C9orf3 | CDCA5 | DDX28 | TSN |
| CAST | COX7C | DMXL1 | ZSWIM6 |
| CCDC43 | CSTF1 | DTYMK | |
| CCPG1 | DAG1 | ECT2 | |
| CDCA5 | DDX27 | FAM63A | |
| CEP55 | DDX28 | FOXK2 | |
| CITED2 | DMXL1 | FPGS | |
| CKS1A | ECT2 | GDPD2 | |
| CLK4 | FPGS | HPGD | |
| COX7C | HCFC2 | KPNA2 | |
| CSTF1 | HN1L | MAPK9 | |
| DAG1 | KIAA1191 | MK1671P | |
| DCUN1D3 | MAPK9 | MYBL2 | |
| DDX27 | MRPS23 | PRPF4 | |
| DDX28 | NUP62 | RGS2 | |
| DMXL1 | PARD6A | SAE1 | |
| DTYMK | PRKDC | SEC24A | |
| EBNA1BP2 | PTGES2 | SECTM1 | |
| ECT2 | PTPN2 | SF3B3 | |
| FAM38A | PTRH2 | SLC2A13 | |
| FAM63A | QTRTD1 | SLC31A2 | |
| FBXO45 | RAP2C | STARD4 | |
| FBXW11 | RGS2 | STMN1 | |
| FOXK2 | RNF112 | TEP1 | |
| FPGS | SECTM1 | TMEM140 | |
| GDPD2 | SF3B3 | TSN | |
| GMPS | SIRT4 | XPO1 | |
| GTPBP4 | SLC2A13 | XPO5 | |
| HCFC2 | SLC31A2 | ZNF608 | |
| HEATR1 | STIP1 | ZSWIM6 | |
| HN1L | TICAM2 | | |
| HPGD | TRUB2 | | |
| KIAA1191 | XPO5 | | |
| KPNA2 | ZNF608 | | |
| LOC729678 | ZSWIM6 | | |
| LRPPRC | | | |
| MAPK9 | | | |
| MCM2 | | | |
| MCM3 | | | |
| MKI67IP | | | |
| MRPS23 | | | |
| MYBL2 | | | |
| NDUFA1 | | | |
| NOL11 | | | |
| NUP62 | | | |
| PAQR4 | | | |
| PARD6A | | | |
| POLE3 | | | |
| PPIP5K2 | | | |
| PRKDC | | | |
| PRPF4 | | | |
| PTGES2 | | | |
| PTPN2 | | | |
| PTRH2 | | | |
| QTRTD1 | | | |
| RAP2C | | | |
| RCC2 | | | |
| RG9MTD1 | | | |
| RGS2 | | | |
| RNF112 | | | |
| RRM2 | | | |
| SAE1 | | | |
| SDCCAG3 | | | |
| SEC24A | | | |
| SEC31A | | | |
| SECTM1 | | | |
| SERPINH1 | | | |
| SF3B3 | | | |
| SIRT4 | | | |
| SLC2A13 | | | |
| SLC31A2 | | | |
| STARD4 | | | |
| STIP1 | | | |
| STMN1 | | | |
| TBC1D9B | | | |
| TEP1 | | | |
| TICAM2 | | | |
| TMEM140 | | | |
| TMEM201 | | | |
| TRUB2 | | | |
| TSN | | | |
| XPO1 | | | |
| XPO5 | | | |
| ZNF608 | | | |
| ZSWIM6 | | | |

Assaying said sample for expression of each of the genes shown in the '90 genes' column of Table 1 provides the advantage of higher AUC. This provides the further advantage of maximised accuracy.

Gene signs and weights are shown in Table 2 below:

**Table 2:**

| **Assay ID** | **Gene Symbol** | **Probe** | **Taqman™ Assay ID** | **Weight** | **Known link with oesophageal dysplasia/adenocarcinoma*** |
|---|---|---|---|---|---|
| 58 | AKR1B10 | NM_020299 | Hs00252524_m1 | -0.74 | yes |
| 30 | APC | NM_000038 | Hs01568269_m1 | -0.79 | yes |
| 80 | ASF1B | NM_018154 | Hs00216780_m1 | 0.71 | no |
| 85 | BID | NM_001196 | Hs00609632_m1 | 0.71 | no |
| 39 | BRCA1 | NM_007300 | Hs01556193_m1 | 0.77 | yes |
| 21 | C9orf3 | AL137535 | Hs00262414_m1 | 0.81 | no |
| 2 | CAST | NM_173060 | Hs00156280_m1 | -0.9 | no |
| 89 | CCDC43 | NM_144609 | Hs00327475_m1 | 0.71 | no |
| 36 | CCPG1 | NM_020739 | Hs00393715_m1 | -0.78 | no |
| 38 | CDCA5 | NM_080668 | Hs00293564_m1 | 0.77 | no |
| 59 | CEP55 | NM_018131 | Hs00216688_m1 | 0.74 | no |
| 55 | CITED2 | NM_006079 | Hs01897804_s1 | -0.74 | no |
| 71 | CKS1A | hCT12654.3 | Custom Assay | 0.72 | no |
| 63 | CLK4 | NM_020666 | Hs00982806_m1 | -0.73 | no |
| 34 | COX7C | NM_001867 | Hs01595219_g1 | -0.78 | no |
| 37 | CSTF1 | NM_001324 | Hs00609730_m1 | 0.77 | no |
| 17 | DAG1 | NM_004393 | Hs00189308_m1 | 0.82 | no |
| 57 | DCUN1D3 | NM_173475 | Hs00708595_s1 | 0.74 | no |
| 32 | DDX27 | NM_017895 | Hs00215471_m1 | 0.79 | no |
| 14 | DDX28 | NM_018380 | Hs00915579_s1 | 0.83 | no |
| 6 | DMXL1 | NM_005509 | Hs00417091_m1 | -0.86 | no |
| 65 | DTYMK | NM_012145 | Hs00992744_m1 | 0.73 | no |
| 83 | EBNA1BP2 | NM_006824 | Hs00199133_m1 | 0.71 | no |
| 13 | ECT2 | NM_018098 | Hs00216455_m1 | 0.84 | no |
| 43 | FAM38A | NM_014745 | Hs00207230_m1 | 0.76 | no |
| 47 | FAM63A | NM_018379 | Hs00218083_m1 | -0.76 | no |
| 78 | FBXO45 | hCT1772149.1 | Hs00397889_m1 | 0.72 | no |
| 41 | FBXW11 | NM_033644 | Hs00606870_m1 | -0.77 | no |
| 68 | FOXK2 | NM_004514 | Hs00895533_m1 | 0.72 | no |
| 16 | FPGS | NM_004957 | Hs00191956_m1 | 0.83 | no |
| 86 | GDPD2 | NM_017711 | Hs00214532_m1 | -0.71 | no |
| 81 | GMPS | NM_003875 | Hs00269500_m1 | 0.71 | no |
| 88 | GTPBP4 | NM_012341 | Hs00202558_m1 | 0.71 | no |
| 10 | HCFC2 | Contig36533_RC | Hs00203344_m1 | -0.84 | no |
| 61 | HEATR1 | NM_018072 | Hs00985319_m1 | 0.74 | no |
| 28 | HN1L | NM_144570 | Hs00375909_m1 | 0.8 | no |
| 48 | HPGD | NM_000860 | Hs00168359_m1 | -0.76 | no |
| 26 | KIAA1191 | NM_020444 | Hs00607464_g1 | -0.8 | no |
| 51 | KPNA2 | NM_002266 | Hs00818252_g1 | 0.75 | no |
| 77 | LOC729678 | hCT2259022 | Hs03678601_g1 | -0.72 | no |
| 87 | LRPPRC | NM_133259 | Hs00370167_m1 | 0.71 | no |
| 40 | MAPK9 | Contig1389_RC | Hs00177102_m1 | -0.77 | no |
| 45 | MCM2 | NM_004526 | Hs01091564_m1 | 0.76 | yes |
| 90 | MCM3 | NM_002388 | Hs00172459_m1 | 0.71 | no |
| 60 | MKI671P | NM_032390 | Hs00757500_s1 | 0.74 | no |
| 7 | MRPS23 | NM_016070 | Hs00608544_m1 | 0.85 | no |
| 52 | MYBL2 | NM_002466 | Hs00942543_m1 | 0.75 | yes |
| 73 | NDUFA1 | NM_004541 | Hs00244980_m1 | -0.72 | no |
| 53 | NOL11 | NM_015462 | Hs00979483_m1 | 0.74 | no |
| 18 | NUP62 | NM_016553 | Hs02621445_s1 | 0.82 | no |
| 69 | PAQR4 | NM_152341 | Hs00373823_m1 | 0.72 | no |
| 3 | PARD6A | NM_016948 | Hs00180947_m1 | 0.93 | no |
| 70 | POLE3 | NM_017443 | Hs00794385_m1 | 0.72 | no |
| 50 | PPIP5K2 | NM_015216 | Hs00274643_m1 | -0.75 | no |
| 24 | PRKDC | NM_006904 | Hs00179161_m1 | 0.81 | no |
| 66 | PRPF4 | NM_004697 | Hs00190796_m1 | 0.73 | no |
| 29 | PTGES2 | NM_025072 | Hs00228159_m1 | 0.79 | no |
| 12 | PTPN2 | NM_080422 | Hs00959886_g1 | 0.84 | no |
| 19 | PTRH2 | NM_016077 | Hs02518444_s1 | 0.82 | no |
| 15 | QTRTD1 | NM_024638 | Hs00226421_m1 | 0.83 | no |
| 5 | RAP2C | AF093744 | Hs00221801_m1 | -0.87 | no |
| 82 | RCC2 | NM_018715 | Hs00603046_m1 | 0.71 | no |
| 44 | RG9MTD1 | NM_017819 | Hs00215145_m1 | 0.76 | no |
| 11 | RGS2 | NM_002923 | Hs00180054_m1 | -0.84 | no |
| 4 | RNF112 | NM_007148 | Hs00246644_m1 | -0.89 | no |
| 74 | RRM2 | Contig41413_RC | Hs01072069_g1 | 0.72 | no |
| 62 | SAE1 | NM_005500 | Hs01062484_g1 | 0.73 | no |
| 84 | SDCCAG3 | NM_006643 | Hs00981269_g1 | 0.71 | no |
| 42 | SEC24A | ENST00000265341 | Hs00378456_m1 | -0.76 | no |
| 46 | SEC31A | NM_016211 | Hs00274601_m1 | -0.76 | no |
| 27 | SECTM1 | NM_003004 | Hs00171088_m1 | -0.8 | no |
| 75 | SERPINH1 | NM_001235 | Hs00241844_m1 | 0.72 | no |
| 9 | SF3B3 | NM_012426 | Hs00418633_m1 | 0.84 | no |
| 25 | SIRT4 | NM_012240 | Hs00202033_m1 | -0.8 | no |
| 8 | SLC2A13 | AK026495 | Hs00369423_m1 | -0.85 | no |
| 23 | SLC31A2 | NM_001860 | Hs00156984_m1 | -0.81 | no |
| 76 | STARD4 | NM_139164 | Hs00287823_m1 | -0.72 | no |
| 35 | STIP1 | NM_006819 | Hs00428979_m1 | 0.78 | no |
| 64 | STMN1 | NM_005563 | Hs01027515_gH | 0.73 | yes |
| 67 | TBC1D9B | NM_015043 | Hs00209268_m1 | -0.72 | no |
| 72 | TEP1 | Contig42649_RC | Hs00200091_m1 | -0.72 | yes |
| 22 | TICAM2 | AB002442 | Hs04189225_m1 | -0.81 | no |
| 49 | TMEM140 | NM_018295 | Hs00251020_m1 | -0.75 | no |
| 79 | TMEM201 | Contig38613_RC | Hs00420510_m1 | 0.71 | no |
| 1 | TRUB2 | NM_015679 | Hs00210383_m1 | 1.0 | no |
| 56 | TSN | Z36850 | Hs00172824_m1 | 0.74 | no |
| 54 | XPO1 | NM_003400 | Hs00418963_m1 | 0.74 | no |
| 20 | XPO5 | NM_020750 | Hs00382453_m1 | 0.82 | no |
| 31 | ZNF608 | AL117587 | Hs00296651_m1 | -0.79 | no |
| 33 | ZSWIM6 | Contig53852_RC | Hs00326109_m1 | -0.78 | no |

| | | | | | |
|---|---|---|---|---|---|
| * "Known link with oesophageal dysplasia/adenocarcinoma" = ascertained by searching "oesophagus"; "oesophageal"; "dysplasia"; "adenocarcinoma" in Pubmed literature database. | | | | | |

It should be noted that the genes are not ranked but are given a weight according to their individual impact on the signature.

Primers such as nucleic acid primers (oligonucleotides) may be designed for the amplification and/or sequencing of any of the genes of interest described herein. Such primers should be of a sufficient length to support their intended use and provide satisfactory specificity to the target sequence and/or a suitable melting point/annealing temperature. Such considerations are well known in the art. Software packages are available to assist in the design of such primers from the sequences referred to herein. This is well within the ambit of the skilled reader. Suitably primers are at least 10nt in length, more suitably 11nt, more suitably 12nt, more suitably 13nt, more suitably 14nt, more suitably 15nt, more suitably 16nt, more suitably 17nt, more suitably 18nt, more suitably 19nt, more suitably 20nt, or even longer. Suitably such primers comprise at least one unnatural nucleotide or nucleotide derivative such as a label or other modification. Suitably such primers are not naturally occurring molecules. Suitably such primers are *in vitro* molecules. Suitably the primers are present in compositions capable of supporting *in vitro* nucleic acid sequence determination and/or *in vitro* amplification.

### COMPUTER IMPLEMENTATION

In so far as the embodiments of the invention described above are implemented, at least in part, using software-controlled data processing apparatus, it will be appreciated that a computer program providing such software control and a storage medium by which such a computer program is stored are envisaged as aspects of the present invention.

Thus the invention provides a method of operating said data processing apparatus, the apparatus set up to execute the method, and/or the computer program itself. The invention also relates to physical media carrying the program such as a computer program product, such as a data carrier, storage medium, computer readable medium or signal carrying the program.

Clearly steps such as providing a sample would be embraced by such a computer program if a software controlled sample handling apparatus was employed. However, if such a step is performed manually at the choice of the operator, then the computer implemented method steps should be understood to comprise or consist of the data processing steps of the method.

### ADVANTAGES OF THE INVENTION

It should be noted that the vast majority of the genes discussed herein have no previous association with dysplasia nor with adenocarcinoma such as oesophageal adenocarcinoma (see table 2 where * "Known link with oesophageal dysplasia/adenocarcinoma" = ascertained by searching "oesophagus"; "oesophageal"; "dysplasia"; "adenocarcinoma" in Pubmed literature database.). Therefore, the association between the vast majority of the genes and the diagnosis (or aiding of the diagnosis) of dysplasia is individually new for each of those genes. These genetic associations provide advantages according to the invention. In one embodiment, suitably the signature comprises one or more genes, suitably 40 genes, having no previous association with dysplasia and/or no previous association with adenocarcinoma such as oesophageal adenocarcinoma. In another embodiment, suitably the signature comprises only genes having no previous association with dysplasia and/or no previous association with adenocarcinoma such as oesophageal adenocarcinoma.

It is an advantage that the 40 gene signature disclosed is entirely transferable between platforms including the Fluidigm™ array.

The genes in the signature could not have been arrived at except through the experiments and insights of the present inventors. There are no known gene signatures in the art directed at aiding the diagnoses discussed herein. In particular, since the overwhelming majority of the genes have never previously been associated with presence of dysplasia, there is no route in the art from the published literature to the present invention.

By careful selection of the tissue samples used, and the analyses conducted, the inventors have been able to identify key diagnostic genes and to describe a robust signature for aiding the diagnosis of dysplasia. Since the start point was the entire human transcriptome comprising some 44,000 RNA transcripts, this is itself a remarkable outcome.

The inventors have used an innovative approach to experimentally pick samples which comprise dysplasia in order to conduct the analysis, rather than simply following the established clinical practice of pathology analysis in order to diagnose patients having dysplasia.

As part of the optimisation of the gene signature of the invention, individual transcripts may be selected on a "per gene" basis in order to optimise the performance. Unless otherwise indicated, the methods of the invention do not require the use of specific transcripts, so any such choice is a matter for the operator.

It is advantage of the invention that the gene signature presented has been designed to perform across all analytical platforms. This involved intellectual effort by the inventors - rather than picking the "best" genes in the signature for a particular analytical platform, the inventors strove to pick genes which performed across multiple platforms. In addition, the inventors went on to test and validate performance of the signature using specially commissioned reagents within different commercially available analytical platforms, thereby arriving at a signature which truly performs across platforms and provides advantages of reliability and robustness in this manner.

It is an advantage of the invention that in the process of designing the gene signatures, different "classifiers" were used in selection of alternate gene groups in order to provide the best possible performance.

As part of the intricate selection process designed by the inventors, candidate genes were ranked according to different analytical platforms such as micro-array results and such as Fluidigm™ results. Intellectual choices were made to select genes for maximum performance across alternate platforms, rather than following conventional approaches such as simply selecting top ranked genes from the test platform.

In arriving at the gene signatures described, the inventors rigorously determined whether differentially expressed genes selected from the initial 44,000 strong data set were useful in diagnosis of dysplasia. The inventors chose not to assume that observation of differential expression would be indicative of performance as a diagnostic. For example, the inventors discovered that some genes showing only very modest differentiation of expression between dysplastic and non-dysplastic samples could in fact be very informative for separation of those samples. Equally, the inventors observed that some very markedly differentially expressed genes did not perform well as separators of dysplastic and non-dysplastic samples. Thus, the inventors carefully chose individual genes for inclusion into the overall signature based on their insights. These insights were based on qualities other than the "size" of the disparity and expression between dysplastic and non-dysplastic samples.

A key advantage delivered by the present invention is that it operates in a platform independent manner. For example, the inventors realised that the use of averaged or weighted average data are better than SVM data for platform independence. Thus, suitably the data are averaged. More suitably the data are weighted average data.

The inventors carefully chose the "classifiers" used to select the pools of genes used in the signatures described herein. For example, whether to use "greedy" classifiers or other classifiers as discussed above. The choices of these classifiers were made in order to improve platform independence and/or to eliminate platform effects from the analyses.

As part of the development of the invention, the inventors made and tested individual platform specific chips such as a fluid IGM chip in order to test and verify the platform independence provided by the invention. This goes beyond normal practice in the art and delivers advantages of platform independent results.

It is an advantage of the invention that a pre-cancerous condition (dysplasia) is diagnosed (or the diagnosis aided). Prior art work in other medical fields has focussed on detection of actual cancer conditions. An advantage of the invention is that dysplasia is detected before it has progressed to cancer.

It is an advantage of the invention that a pathological state is diagnosed (or it's diagnosis is aided) i.e. the presence of dysplasia is diagnosed (or it's diagnosis is aided).

Other approaches in other medical fields have tended to focus on the prediction of prognosis. By diagnosing (or aiding diagnosis of) a pathological state, treatment is facilitated.

It is an advantage of the invention that the combination of genes in the signature provides a powerful readout/diagnostic tool. In this regard, it is very surprising that a dominant set of genes within the large group analysed did not emerge. It would be expected in the art that a sub-group of dominant genes would be responsible for the majority of the observed effect. It was therefore very surprising to the inventors that the contribution/value of each of the genes in the signature is very evenly distributed. This provides the advantage of the results of the analysis being less disturbed by weighting towards a small number of dominant genes.

It was surprising to the inventors that an unpredictable set of genes for the signature was identified. By "unpredictable" it is meant, for example, that the great majority of the genes identified have no known relationship to cancer. This is very surprising, since it would expected that genes previously linked to cancer would have dominated the analysis.

It is surprising to the inventors that unsuspected signalling pathways contributed numerous genes to the signatures disclosed. Thus, the genes in the signatures would never have been expected by a skilled reader in the absence of the disclosures herein.

It is an advantage of the invention that the gene signature may be used across all platforms unusually smoothly.

The inventors diverged from conventional practice by eschewing clinical relevance as a factor in selecting the genes in the signature. For example, a typical prior art approach might be to sort candidate genes by clinical relevance. The genes having greatest clinical relevance would then be selected for further study. By contrast, the present inventors departed from the beaten track by investing a greater weight into their methodology rather than by following prior art approaches of weighting by clinical relevance. This is not a usual way to conduct this type of experimental science. This unconventional approach has delivered an advantageous gene signature useful in diagnosis, or aiding diagnosis, of dysplasia.

It is an advantage of the invention that the results delivered are more reliable than those delivered by consensus histopathology. For example, we have calculated Kappa values that compare the classification given by the gene signature with that given by the pathologist who classified the samples for the experiment. These Kappa values are all greater than 0.5. In comparison, two published papers that compare the performances of pathologists in this area give Kappa values for their agreement of 0.14 and 0.5. Thus it is clear from the Kappa statistics that our test is more reproducible than pathologist for the diagnosis of dysplasia.

The main advantage of the signature of the invention is to provide a more robust diagnosis of the prevalent grade of dysplasia, particularly for low grade dysplasia cases which are difficult to grade. This is a highly relevant clinical question given that low grade dysplasia is now an intervention point. In clinical practice it is a problem to ensure that the diagnosis at a given point in time is accurate in order to avoid under or over treatment. The present invention provides a solution to this problem.

### FURTHER APPLICATIONS

In one aspect, the invention relates to a method of diagnosing dysplasia in a subject, such as in the oesophagus of a subject, by carrying out the method steps as described above.

In one aspect, the invention relates to a method of obtaining information useful in the diagnosis of dysplasia in a subject, such as in the oesophagus of a subject, by carrying out the method steps as described above.

In one aspect, the invention relates to a method of detecting dysplasia in a subject, such as in the oesophagus of a subject, by carrying out the method steps as described above.

In one aspect, the invention relates to a method of determining the presence of dysplasia in a subject, such as in the oesophagus of a subject, by carrying out the method steps as described above.

In one aspect, the invention relates to a method of identifying a subject having dysplasia, such as dysplasia in the oesophagus, by carrying out the method steps as described above.

The invention provides a method of treating dysplasia in a subject, the method comprising performing the method as described above wherein if the presence of dysplasia in said subject is indicated, then one or more of radio frequency ablation treatment, argon plasma coagulation, photodynamic therapy, cryotherapy or endoscopic mucosal resection is administered to said subject. Most suitably radio frequency ablation treatment is administered to said subject.

Further particular and preferred aspects are set out in the accompanying independent and dependent claims. Features of the dependent claims may be combined with features of the independent claims as appropriate, and in combinations other than those explicitly set out in the claims.

Where an apparatus feature is described as being operable to provide a function, it will be appreciated that this includes an apparatus feature which provides that function or which is adapted or configured to provide that function.

### BRIEF DESCRIPTION OF THE DRAWINGS

Embodiments of the present invention will now be described further, with reference to the accompanying drawings, in which:
Figure 1 shows density distributions of the 90 deltaCt values for all samples in an experiment, Solid line = NDBE, Dashed line = HGD, Black = Run 1, Red = Run 2, Green = Run3, Blue = Run4.
Figure 2 shows ROC curve for unnormalised data (red - lower) and Gaussian normalised data (black - upper)
Figure 3 shows a plot showing how the Area under the ROC curve (AUC) varies as the number of genes in the gene signature is increased.
Figure 4 shows a boxplot of the AUC performance of random selections of genes of from the list of 90 genes.
Figure 5 shows Youden's statistics for 40 gene signature.
Figure 6: Study overview. NDBE: Non-dysplastic Barrett's esophagus, LGD: Low grade dysplasia,
   HGD: High grade dysplasia, EA: Esophageal adenocarcinoma
Figure 7 A. Separation of NDBE from HGD consensus samples by the 90-gene signature using the microarray dataset as a training set. B. Gene Signature separating the untrained samples on microarray dataset. NDBE: Non-dysplastic Barrett's esophagus, LGD: Low grade dysplasia, HGD: High grade dysplasia, EA: Esophageal adenocarcinoma
Figure 8. A. Validation of the 90-gene signature on esophageal samples from different stages of disease progression. B. ROC curve for NDBE versus LGD, HGD and EA. C. ROC curve for NDBE versus LGD. D. ROC curve for NDBE versus HGD. NDBE: Non-dysplastic Barrett's esophagus, ID: Indefinite for dysplasia, LGD: Low grade dysplasia, HGD: High grade dysplasia, EA: Esophageal adenocarcinoma
Figure 9: Proposed clinical use of the 90-gene signature
Figure 10 (Supplementary Figure 1). Plot showing how the area under the receiver operator curve (AUC) varies as the number of genes in the gene signature is increased. Genes were ranked by p-value from a Limma analysis and leave-one-out cross-validation was used.

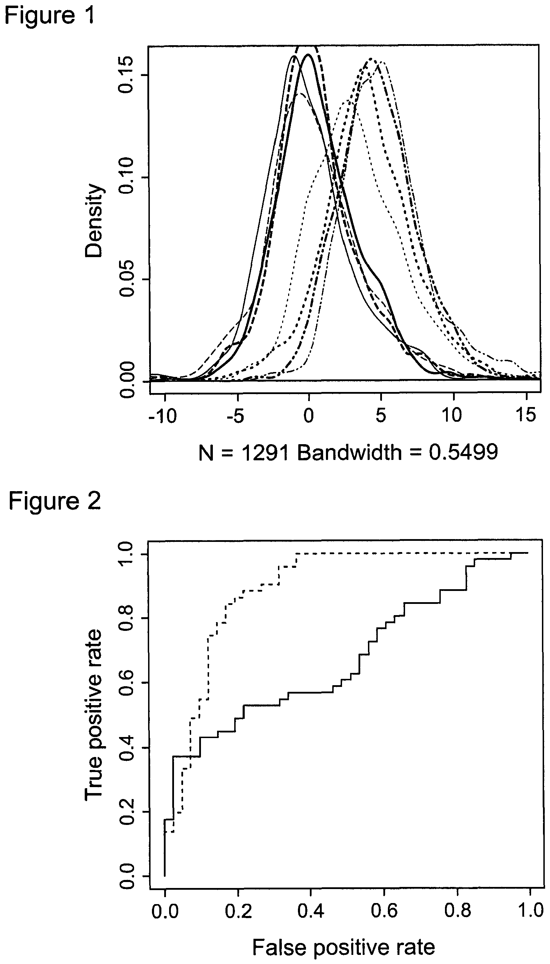

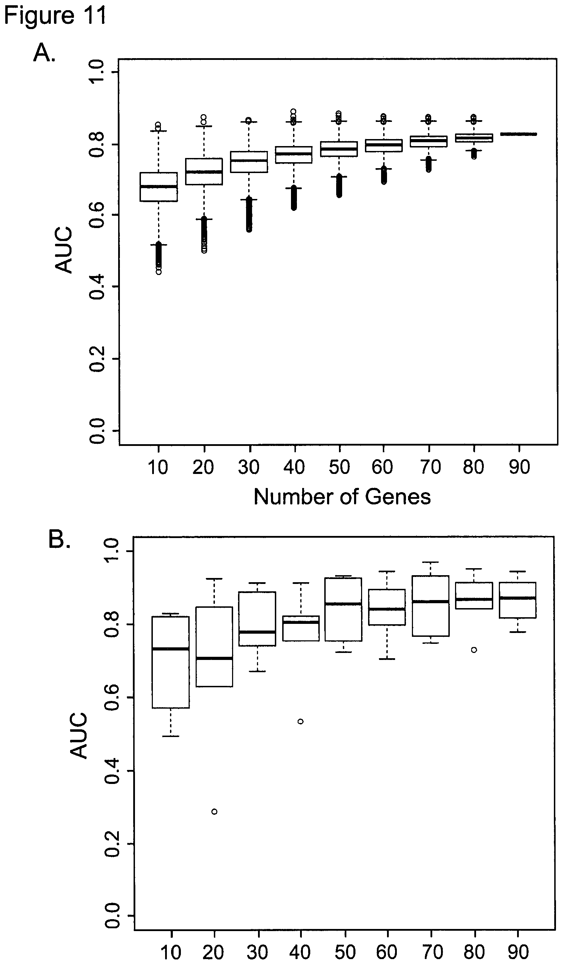

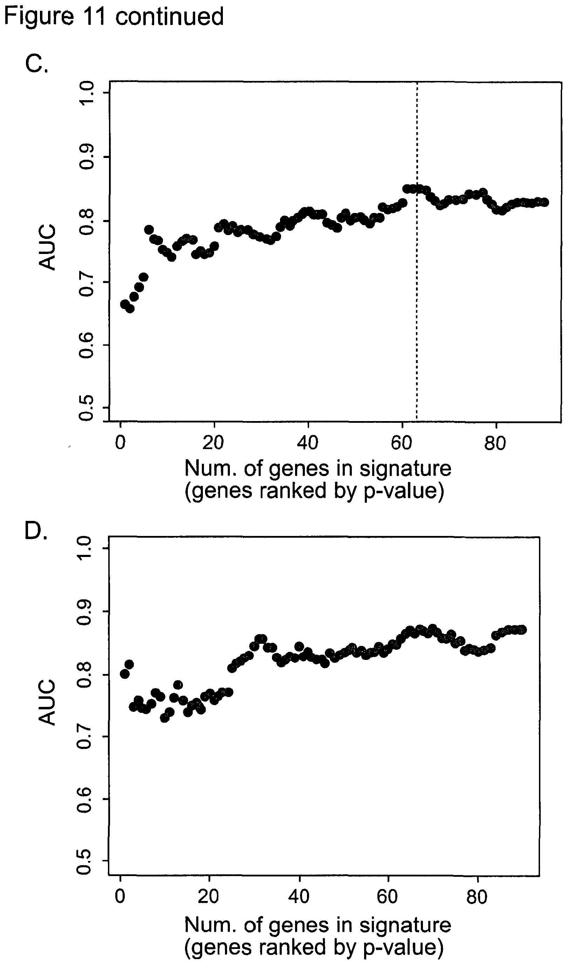

Figure 11 (Supplementary Figure 2): Justification for the number of genes in the signature. (A) A comparison of the AUC for randomly selected signatures of different sizes from the Fluidigm validation data. (B) Repeating the analysis of panel A six times, leaving out one of the datasets each time. For each signature size we record the gene combination that gives the highest AUC. We then apply these 'best' gene signatures to the "leave one out" data. The resulting AUCs are plotted in the figure. (C) We ranked the 90 probes according to their differential expression between NDBE and HGD using the R package Limma. We started with a singleton signature composed of the top ranked probe, calculated the AUC, then added the next ranked probe and recalculated the AUC, etc. The figure shows a plot of AUC as the length of the signature is increased. The dotted line demarks the point at which AUC remains stable (D) As for panel C but employing cross-validation. We leave out a dataset, use Limma to analyse the remaining datasets to rank the genes. Then apply gene signatures of increasing length to the "leave one out" dataset. The figure shows how the average AUC varies with increasing signature length.
Figure 12 (Supplementary Figure 3). Validation of gene signature on external datasets. A. Greenawalt et al dataset where 55 out of 90 probes mapped and B. Wang et al where 39 out of 90 probes mapped
Figure 13 (Supplementary Figure 4). Kaplan-Meir curve of individuals diagnosed with NDBE classified to low- or high-risk using the 90-gene signature.
Figure 14 shows comprehensive data for the 40 gene signature of table 1.

### DESCRIPTION OF THE EMBODIMENTS

Although illustrative embodiments of the invention have been disclosed in detail herein, with reference to the accompanying drawings, it is understood that the invention is not limited to the precise embodiment and that various changes and modifications can be effected therein by one skilled in the art without departing from the scope of the invention as defined by the appended claims and their equivalents.

### Example 1: Gaussian Normalisation

Even after control probe normalisation intensity values between different runs are not always comparable (see Figure 1). There can be a shift in the mean intensity, in particular between Runs 1 & 4 compared to Runs 2 & 3.

Such a shift might be corrected for, for example by using a calibration sample.

Alternatively the shift might be corrected for using a data analysis solution. The solution that we show in this example is to Gaussian normalise the arrays. To Gaussian normalise, firstly the 40 (or 90) values were ranked and the rank divided by 41 (or 91). These values were then taken to be a vector of probabilities from a Gaussian distribution and converted to variables using the distribution's quantile function.

That is, if *rᵢ* is the rank of the *i*th probe on the array, its value is Gaussian transformed to *xᵢ* where Pr(X < *xᵢ*) = *rᵢ*=41 (or 91), and *xᵢ* are assumed to be normally distributed.

ROC curves for the unnormalised data (red) and the Gaussian normalised data (black) are shown in Figure 2 for a 90 gene signature.

Thus it can be seen that Gaussian normalisation has greatly improved the AUC which has gone from 0.66 to 0.90. It is important to note that it is a normalisation method that can be applied to each and any array in isolation, it does not depend on distributional information from any prior array experiments, so would be readily applicable in clinical practice. Thus the normalisation method using Gaussian normalisation delivers specific technical advantages when optionally included in the methods described herein.

### Example 2A: 40 Gene Signature

The 40 gene signature provides the advantage of delivering clinically reliable information or a clinically reliable indication. Use of fewer genes in the analysis results in information of clinically questionable relevance.

In particular, one conclusion which can be supported using the 40 gene signature taught herein is whether or not the subject has dysplasia such as oesophageal dysplasia. Use of fewer than 40 genes does not reliably support this type of conclusion.

Another advantage of the 40 gene signature is the high value area under the curve ('AUC', which measures the diagnostic accuracy of a panel), which it provides. At 40 genes, the AUC is above 90% as shown in Figure 3.

In other words, Figure 3 clearly demonstrates that the area under the curve (AUC) for the 40 gene signature is equivalent to that for the 90 gene signature. The area under the curve indicates the proportion of samples that would be correctly classified into low or high risk based on the signature.

Figure 3 clearly shows that the AUC decreases below 40 genes. This is very marked as the AUC decreases below 90% for signatures below 35 genes. In addition, this list is specific for that combination of 40 genes.

Further evidence of the effectiveness of the 40 gene signature is provided:
When we use a clinically useful cut-off for a sensitivity of 0.825 for our data then the specificity is the same for the 40 gene signature and the expanded 90 gene signature:

| | Sensitivity | Specificity |
|---|---|---|
| 90 genes: | 0.825 | 0.784 |
| 40 genes: | 0.825 | 0.784 |

If however we look at a perfect sensitivity (sensitivity =1) then the 90 gene signature would outperform the 40 gene signature:

| | Sensitivity | Specificity |
|---|---|---|
| 90 genes: | 1 | 0.157 |
| 40 genes: | 1 | 0.059 |

This outperformance at perfect sensitivity may be understood as an advantage for choosing the 90 gene signature, but for clinical utility the 40 gene signature is surprisingly good enough i.e. at a sensitivity of 82.5% as shown above. Thus the utility of the 40 gene signature is demonstrated.

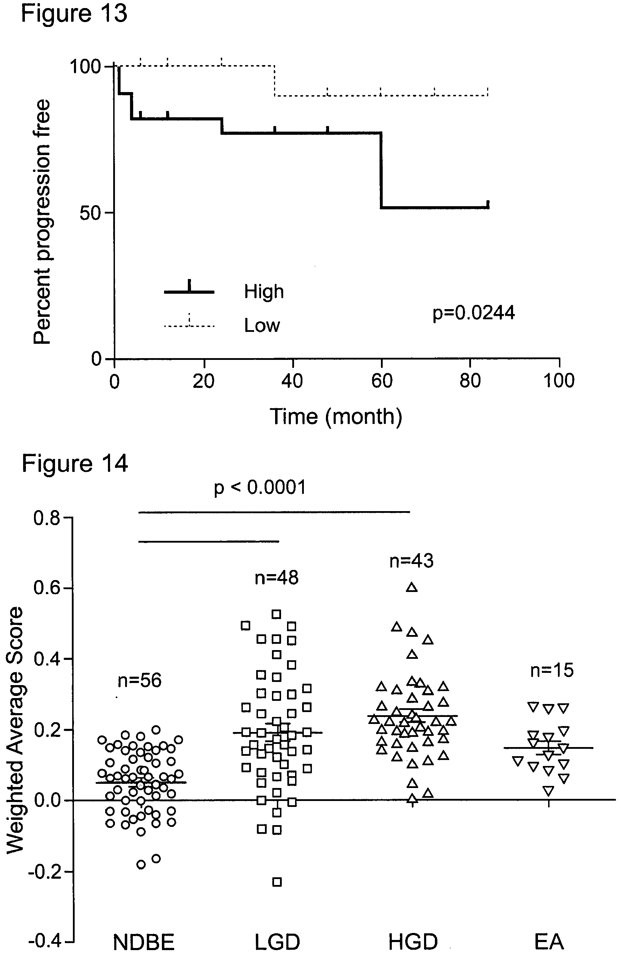

In addition, we refer to Figure 14 which shows a comprehensive data set for the 40 gene signature of Table 1.

### Example 2B: Application to Subjects

We provide the following outline of applying the test to a patient/subject:
A biopsy sample is taken (or provided) from the Barrett's oesophagus segment of a patient.

The mRNA from the sample is extracted and processed and the expression levels of 40 specific genes shown in table 1 is assessed on this sample.

The gene expression levels are then normalised and a weighted average score is calculated based on the expression of these 40 genes which gives different pre-set weights for each of the genes.

Based on the weighted average score the sample would be assigned as 'high risk' or 'low risk' for dysplasia based on whether the weighted average score is above or below a threshold value of zero.

### Example 3: 40 Gene Signature - Gene Identities

As shown in figure 4, the performance of gene signatures from randomly selected perform generally below the acceptable threshold of 0,88 AUC. As the size of the signature increases, the chance of finding an acceptable signature increases but still does not reach the AUC of 0.96 obtained with the optimal signature.

Thus we demonstrate that the particular identity of the 40 genes selected and presented herein as a single signature contributes a special qualitative advantage to the invention.

As Figure 4 demonstrates, a conventional approach such as adding genes to add weight to the analysis still does not deliver an AUC as high as the 40 gene signature of the invention. Thus it is shown that the 40 gene signature of the invention possesses a special technical benefit which could have been predicted, and would not have been arrived at following conventional approaches in the art.

### Example 4: Method Of Aiding The Diagnosis Of Dysplasia

A method of aiding the diagnosis of dysplasia in a subject is carried out, said method comprising:
(a) providing a sample from the oesophagus of said subject;
   In this example, the sample is an *in vitro* biopsy previously obtained from the oesophagus of the subject.
(b) assaying said sample for expression of each of the genes shown in the '40 genes' column of Table 1;
   In this example, the RNA is extracted from the biopsy. This is reverse transcribed into cDNA. Taqman™ gene expression assays were used to assess gene expression.
(c) normalising the expression levels of the genes in part (b) to expression levels of reference gene(s) from a non-dysplastic sample;
   In this example, three reference genes are assessed for each sample (RPS18, POLR2A, GAPDH) to normalise for cDNA input. For each sample the median of the three reference genes' Ct values is used to calculate the ΔCt values for the 40 target genes (i.e. Ct of target gene - median Ct of reference genes).

Each sample's 40 ΔCt values are then Gaussian normalized. That is, if ri is the rank of the ith probe on the array, its value is Gaussian transformed to xi where Pr(X < xi) = ri/41, and xi are assumed to be distributed according to a standard Gaussian.
(d) determining from the normalised expression levels of (c) a gene signature score for the sample, wherein a gene signature score greater than a reference threshold indicates presence of dysplasia in said subject.

In this example, each sample's gene signature score is calculated by taking a weighted average of the 40 normalized ΔCt values. In this example, the weights being the normalized t-statistics from the training data Limma analysis; weights ranged in absolute value from 1 to 0.501 (table 2).

In this example, the values for the reference threshold used were:
>> sensitivity = 1.000
>> specificity = 0.059
>> **threshold** = **-0.409**

In another example, for better specificity the reference threshold used were:
>> sensitivity = 0.825
>> specificity = 0.784
>> **threshold = -0.124**

### Example 5: Gene Signature for Diagnosis of Dysplasia

**BACKGROUND & AIMS:** The histopathological diagnosis of dysplasia dictates management decisions for patients with Barrett's esophagus (BE). This is despite profound intra- and inter-observer variability in assigning a diagnosis, particularly for low-grade dysplasia (LGD). We aimed to identify a biomarker which could assign patients with LGD to a low- or high-risk group.

**METHODS:** A gene expression signature for identifying high-risk BE was developed using stringently graded samples (n=28 non-dysplastic BE, 23 dysplastic and 8 esophageal adenocarcinoma (EA)). Pathway analysis of the resulting gene-set was performed using MetaCore (GeneGo Inc). The signature was validated in two publically available datasets and in an independent cohort of samples, including LGD (n=169).

**RESULTS:** A 90-gene signature separated non-dysplastic BE from high-grade dysplasia (p<0.0001). Pathway analysis revealed that the RAN (RAs-related Nuclear protein) regulation pathway was the main contributor to this gene-set (p<0.0001) and the transcription factor c-MYC regulated at least 30% of genes within the signature (p<0.0001). In externally published datasets, the signature separated non-dysplastic BE samples from EA samples (p=0.0012). In an independent validation cohort, the signature predicted the dysplasia status of Barrett's samples with an AUC of 0.87 (95% CI, 0.82-0.93). 64% of LGD samples fell into the high-risk category which was correlated with a higher progression rate (p=0.047).

**CONCLUSIONS:** Our 90-gene signature can objectively assign patients to a low- or a high-risk category. This tool has the potential to be an adjunct to histopathological analysis for high-risk BE. This will be most beneficial for patients without a definitive evidence of high-grade dysplasia but for whom early endoscopic intervention is warranted.

### Patients and Methods

### Microarray gene expression profiling (Training set)

Fresh frozen esophageal samples (n=150) were obtained between 2000 and 2006 from four centres in the United Kingdom: Cambridge University Hospitals NHS Foundation Trust, Cambridge; University College London Hospitals NHS Foundation Trust, London; Foundation Trust, Bristol; Gloucestershire Hospitals NHS Foundation Trust, Gloucester. All samples were taken following endoscopy or surgery from consented patients at different stages of Barrett's neoplastic progression following approval by local ethical committees. A frozen section from each frozen sample used for molecular profiling was taken for consensus histopathological reporting by two expert gastrointestinal pathologists blinded to the diagnosis of the corresponding clinical biopsies. Samples were graded for dysplasia and cancer using the Vienna histological classification ⁵. In their review the pathologists also correlated the frozen section with the corresponding clinical FFPE H&E sample to aid the diagnosis. Samples with at least 50% of the epithelial cells displaying the diagnosis of interest, were taken forward (Figure 6). mRNA was extracted using the PicoPure® RNA isolation kit (Applied Biosystems®) according to manufacturer's instructions. mRNA that passed the quality control (A260/A280 ratio > 1.8; A260/A230 ratio > 1.6) was amplified using MessageAmp™ II kit (Life Technologies™). After *in vitro* transcription the antisense RNA was purified using the MinElute® kit (Qiagen). Five micrograms of each sample and control were labelled with cyanine dyes (Cy-3 or Cy-5) and hybridized to complementary gene-specific probes on a custom Agilent microarray (44K 60-mer oligo-microarray, Agilent Technologies, Santa Clara, CA, USA). Each sample was hybridized twice using a dye reversal strategy. The images were then scanned and the fluorescence intensities for each probe recorded. After normalisation using the Universal Human Reference RNA (UHRR) and correction of array intensity data, the ratios of transcript abundance (experimental to control) were obtained. A de-trending template comprised of 470 reporter probes was used to remove data bias.

### Generation of Gene Signature

The categories of non-dysplastic BE (NDBE) and HGD were used to identify a classifier for dysplasia as they are the most clear-cut histopathological diagnoses. As there was considerable variability in the reporting of LGD and since EA is a late stage with variable differentiation status which might confound gene expression - both these groups were eliminated from the training set but were used later to validate the classifier. This resulted in 28 NDBE and 13 HGD samples being used as a training set. For each sample in the microarray data, log intensities were scale and location normalized using the median and mad values for that array. All analyses were carried out using the R statistical environment (R Development Core Team 2013)²².

The NDBE and HGD arrays were analysed for differential expression using the R package Limma²³. The genes were ordered by the moderated t-statistic which were recorded as weights and then normalised so that the highest weight equalled 1.

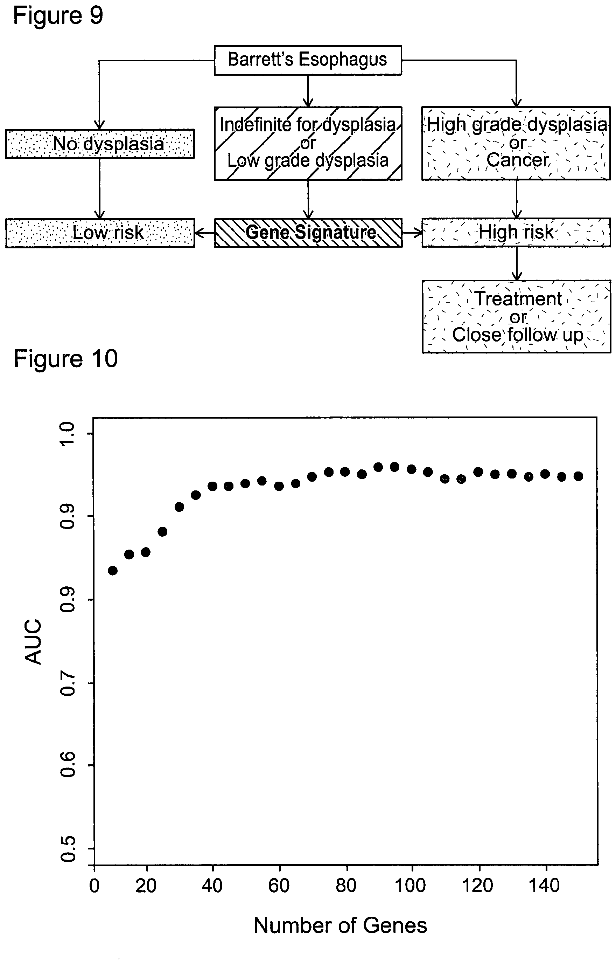

Gene signatures of different lengths n were investigated successively. Leave-one-out cross-validation (LOOCV) was used, that is one array was left out, then the remaining 40 samples were put through a Limma analysis and all genes on the array ranked by p-value. For a signature of length n the top ranked n genes were selected. This signature was used to calculate a score for the left out array, based on the weighted average of the expression of the n genes on the left out array. This was repeated for all 41 arrays to give 41 scores and the area under the receiver operator curve (AUC) calculated. This was repeated for successive values of n. Figure 10 (Supplementary Figure 1) plots the AUC values against n. The AUC increases with increasing number of genes in the signature, the maximum AUC occurring at 90 genes. The steps used to justify the signature size and to address possible over-fitting are presented in Figure 11 (supplementary Figure 2).

To determine the classification method a number of different classifiers including k-nearest neighbors, diagonal linear discriminant analysis and support vector machine (SVM) were compared to classification using the simple weighted-average of values. Analysis was performed using the R package CMA²⁴. This confirmed that a weighted average of values performed adequately when compared to the more sophisticated methods. Whilst other methods, for example SVM, did perform slightly better than a weighted-average in cross-validation, the difference was not large. Moreover, methods like SVM can be prone to over-fitting and therefore the weighted-average classification method was chosen.

### Pathway analysis

Functional pathways of the genes on the gene-signature were analysed using the MetaCore analysis suite (GeneGo Inc: http://www.genego.com). The genes were entered into the analysis programme based on the direction of gene regulation within the signature (i.e. up- or down-regulated). Transcription factors involved in the regulation of these genes were analysed in the same way.

### Validation sets

Publically available microarray datasets containing gene-expression data on Barrett's esophagus and esophageal adenocarcinoma samples were accessed through NCBI GEO (Gene Expression Omnibus). A separate set of samples (n=169) for validation were collected retrospectively from patients consented at the Cambridge University Hospitals NHS Foundation Trust, Cambridge, UK and the Academic Medical Center, Amsterdam, Netherlands following local ethical approval (Table A). These samples were obtained during endoscopy and snap frozen immediately after collection. A section from each frozen sample was taken for histopathological diagnosis by an expert gastrointestinal pathologist. Samples with at least 20% of the section displaying the diagnosis of interest were taken forward. The samples in the validation set were completely independent from the samples in the training set.

**Table A. Demographic data for validation samples**

| **Diagnosis** | **Number** | **Age** | | **BE Length** | **Follow up duration** |
|---|---|---|---|---|---|
| | | Mean (Range) years | (M:F) | Mean (Range) cm | Mean (Range) years |
| NDBE | 51 | 64.5 (34-86) | 2.4:1 | 6.67 (2-14) | 5.14 (0-17) |
| LGD | 28 | 70.52 (58-89) | 6:1 | 7.68 (2-16) | 3.16 (0-11) |
| ID | 13 | 66.08 (41-78) | 12:1 | 6.82 (4-11) | 1.85 (0-11) |
| HGD | 36 | 69.42 (32-87) | 8:1 | 8.3 (1-12) | 3.44 (0-6) |
| EA | 32 | | | | |
| Duodenum | 9 | | | | |

| | | | | | |
|---|---|---|---|---|---|
| NDBE: Non-dysplastic Barrett's esophagus, ID: Indefinite for dysplasia, LGD: Low grade dysplasia, HGD: High grade dysplasia, EA: Esophageal adenocarcinoma | | | | | |

### RNA extraction, reverse transcription and qRT-PCR

Total RNA was extracted using the miRNeasy® Mini Kit (Qiagen, Hilden, Germany) following manufacturer's instructions. RNA concentration and quality were measured using the Nanodrop ND-1000 spectrophotometer (Peqlab, Erlangen, Germany) and stored at -80°C. Reverse transcription to cDNA was done using 1µg of RNA with acceptable quality (A260/A280 ratio > 1.8) using QuantiTech® reverse transcription kit (Qiagen, Hilden, Germany) according to manufacturer's instructions. Taqman® gene expression assays were used to assess gene expression levels (Life Technologies™). qPCR was performed on the BioMark™ real time PCR system using the microfluidic 96.96 dynamic array chip (Fluidgm®, San Francisco, USA) 24. Multiplexed pre-amplification of the targets was done using the Specific Target Amplification protocol by Fluidigm® where the cDNA was pre-amplified with a 0.2X pool of the Taqman® gene expression assays and Taqman® PreAmp master mix for 14 cycles.

Three reference genes were included (*RPS18, POLR2A, GAPDH*) to normalise for cDNA input. For each sample the median of the three reference genes' Ct values was used to calculate the ΔCt values for the 90 target genes (i.e. Ct of target gene - median Ct of reference genes). Each sample's 90 ΔCt values were then Gaussian normalized. That is, if rᵢ is the rank of the *i*^{th} probe on the array, its value is Gaussian transformed to xᵢ where Pr(X < xᵢ) = rᵢ/91, and xᵢ are assumed to be distributed according to a standard Gaussian. Each sample's gene signature score was calculated by taking a weighted average of the 90 normalized ΔCt values, the weights being the normalized t-statistics from the training data Limma analysis; weights ranged in absolute value from 1 to 0.501.

### Results

### Identification of the Gene Signature

Using a leave-one-out cross-validation (LOOCV) analysis which minimises over-fitting of the data an optimal set of 90 genes (Supplementary table 1) was identified.

**Supplementary Table 1: 90-Gene Signature with Taqman® Assay ID**

| **No.** | **Gene Symbol** | **Gene Name** | **Assay ID (Taqman)** |
|---|---|---|---|
| 1 | TRUB2 | TruB pseudouridine (psi) synthase homolog 2 (E. coli),Gene hCG18556 Celera Annotation | Hs00210383_m1 |
| 2 | CAST | calpastatin, Gene hCG2016526 Celera Annotation | Hs00156280_m1 |
| 3 | PARD6A | par-6 partitioning defective 6 homolog alpha (C. elegans), Gene hCG2025821 Celera Annotation | Hs00180947_m1 |
| 4 | RNF112 | ring finger protein 112, Gene hCG30688 Celera Annotation | Hs00246644_m1 |
| 5 | RAP2C | RAP2C, member of RAS oncogene family,Gene hCG2043125 Celera Annotation | Hs00221801_m1 |
| 6 | DMXL1 | Dmx-like 1, Gene hCG2028156 Celera Annotation | Hs00417091_m1 |
| 7 | MRPS23 | mitochondrial ribosomal protein S23, Gene hCG32232 Celera Annotation | Hs00608544_m1 |
| 8 | SLC2A13 | solute carrier family 2 (facilitated glucose transporter), member 13, Gene hCG38260 Celera Annotation | Hs00369423_m1 |
| 9 | SF3B3 | splicing factor 3b, subunit 3, 130kDa, Gene hCG1998533 Celera Annotation | Hs00418633_m1 |
| 10 | HCFC2 | host cell factor C2,Gene hCG20897 Celera Annotation | Hs00203344_m1 |
| 11 | RGS2 | regulator of G-protein signaling 2, 24kDa, Gene hCG41052 Celera Annotation | Hs00180054_m1 |
| 12 | PTPN2 | protein tyrosine phosphatase, non-receptor type 2, Gene hCG1999834 Celera Annotation | Hs00959886_m1 |
| 13 | ECT2 | epithelial cell transforming sequence 2 oncogene, Gene hCG1811567 Celera Annotation | Hs00216455_m1 |
| 14 | DDX28 | DEAD (Asp-Glu-Ala-Asp) box polypeptide 28, Gene hCG1643348 Celera Annotation | Hs00915579_m1 |
| 15 | QTRTD1 | queuine tRNA-ribosyltransferase domain containing 1, Gene hCG18874 Celera Annotation | Hs00226421_m1 |
| 16 | FPGS | folylpolyglutamate synthase, Gene hCG18548 Celera Annotation | Hs00191956_m1 |
| 17 | DAG1 | dystroglycan 1 (dystrophin-associated glycoprotein 1), Gene hCG20125 Celera Annotation | Hs00189308_m1 |
| 18 | NUP62 | nucleoporin 62kDa, Gene hCG19665 Celera Annotation | Hs02621445_s1 |
| 19 | PTRH2 | peptidyl-tRNA hydrolase 2, Gene hCG1775207 Celera Annotation | Hs02518444_s1 |
| 20 | XPO5 | exportin 5, Gene hCG19013 Celera Annotation | Hs00382453_m1 |
| 21 | C90rf3 | chromosome 9 open reading frame 3, Gene hCG2003660 Celera Annotation | Hs00262414_m1 |
| 22 | TICAM2 | toll-like receptor adaptor molecule 2 | Hs04189225_m1 |
| 23 | SLC31A2 | solute carrier family 31 (copper transporters), member 2, Gene hCG29184 Celera Annotation | Hs00156984_m1 |
| 24 | PRKDC | protein kinase, DNA-activated, catalytic polypeptide, Gene hCG1983728 Celera Annotation, Gene hCG1811085 Celera Annotation | Hs00179161_m1 |
| 25 | SIRT4 | sirtuin 4, Gene hCG27774 Celera Annotation | Hs00202033_m1 |
| 26 | KIAA1191 | KIAA1191, Gene hCG40787 Celera Annotation | Hs00607464_g1 |
| 27 | SECTM1 | secreted and transmembrane 1, Gene hCG1773686 Celera Annotation | Hs00171088_m1 |
| 28 | HN1L | hematological and neurological expressed 1-like, Gene hCG1988476 Celera Annotation | Hs00375909_m1 |
| 29 | PTGES2 | prostaglandin E synthase 2, Gene hCG1785478 Celera Annotation | Hs00228159_m1 |
| 30 | APC | adenomatous polyposis coli, Gene hCG2031476 Celera Annotation | Hs01568269_m1 |
| 31 | ZNF608 | zinc finger protein 608 | Hs00296651_m1 |
| 32 | DDX27 | DEAD (Asp-Glu-Ala-Asp) box polypeptide 27, Gene hCG1810935 Celera Annotation | Hs00215471_m1 |
| 33 | ZSWIM6 | zinc finger, SWIM-type containing 6, Gene hCG18020 Celera Annotation | Hs00326109_m1 |
| 34 | COX7C | cytochrome c oxidase subunit VIIcGene hCG37008 Celera Annotation | Hs01595219_g1 |
| 35 | STIP1 | stress-induced-phosphoprotein 1, Gene hCG21368 Celera Annotation | Hs00428979_m1 |
| 36 | CCPG1 | cell cycle progression 1, Gene hCG40050 Celera Annotation, Gene hCG2042696 Celera Annotation | Hs00393715_m1 |
| 37 | CSTF1 | cleavage stimulation factor, 3' pre-RNA, subunit 1, 50kDa, Gene hCG39097 Celera Annotation | Hs00609730_m1 |
| 38 | CDCA5 | cell division cycle associated 5, Gene hCG23394 Celera Annotation | Hs00293564_m1 |
| 39 | BRCA1 | breast cancer 1, early onset, Gene hCG16943 Celera Annotation | Hs01556193-m1 |
| 40 | MAPK9 | mitogen-activated protein kinase 9, Gene hCG1984637 Celera Annotation | Hs00177102_m1 |
| 41 | FBXW11 | F-box and WD repeat domain containing 11, Gene | Hs00606870_m1 |
| | | hCG37596 Celera Annotation | |
| 42 | SEC24A | SEC24 family, member A (S. cerevisiae)Gene hCG1981418 Celera Annotation | Hs00378456_m1 |
| 43 | FAM38A | family with sequence similarity 38, member A,Gene hCG1980844 Celera Annotation | Hs00207230_m1 |
| 44 | RG9MTD1 | RNA (guanine-9-) methyltransferase domain containing 1, Gene hCG39275 Celera Annotation | Hs00215145_m1 |
| 45 | MCM2 | minichromosome maintenance complex component 2, Gene hCG39269 Celera Annotation | Hs01091564_m1 |
| 46 | SEC31A | SEC31 homolog A (S. cerevisiae), Gene hCG20214 Celera Annotation | Hs00274601_m1 |
| 47 | FAM63A | family with sequence similarity 63, member A, Gene hCG1778763 Celera Annotation | Hs00218083_m1 |
| 48 | HPGD | hydroxyprostaglandin dehydrogenase 15-(NAD), Gene hCG39037 Celera Annotation | Hs00168359_m1 |
| 49 | TMEM140 | transmembrane protein 140, Gene hCG2014222 Celera Annotation | Hs00251020_m1 |
| 50 | PPIP5K2 | diphosphoinositol pentakisphosphate kinase 2, Gene hCG38349 Celera Annotation | Hs00274043_m1 |
| 51 | KPNA2 | karyopherin alpha 2 (RAG cohort 1, importin alpha 1), Gene hCG2039660 Celera Annotation | Hs00818252_g1 |
| 52 | MYBL2 | v-myb myeloblastosis viral oncogene homolog (avian)-like 2, Gene hCG38470 Celera Annotation | Hs00942543_m1 |
| 53 | NOL11 | nucleolar protein 11, Gene hCG1987243 Celera Annotation | Hs00979483_m1 |
| 54 | XPO1 | exportin 1 (CRM1 homolog, yeast), Gene hCG1986857 Celera Annotation | Hs00418963_m1 |
| 55 | CITED2 | Cbp/p300-interacting transactivator, with carboxy-terminal domain, 2, Gene hCG32930 Celera Annotation | Hs01897804_s1 |
| 56 | TSN | translin,Gene hCG37642 Celera Annotation | Hs00172824_m1 |
| 57 | DCUN1D3 | DCN1, defective in cullin neddylation 1, domain containing 3 (S. cerevisiae), Gene hCG38244 Celera Annotation | Hs00708595_s1 |
| 58 | AKR1B10 | aldo-keto reductase family 1, member B10 (aldose reductase), Gene hCG20345 Celera Annotation | Hs00252524_m1 |
| 59 | CEP55 | centrosomal protein 55kDa, Gene hCG39533 Celera Annotation | Hs00216688_m1 |
| 60 | MKI67IP | MKI67 (FHA domain) interacting nucleolar phosphoprotein, Gene hCG1750014 Celera Annotation | Hs00757500_s1 |
| 61 | HEATR1 | HEAT repeat containing 1, Gene hCG25461 Celera Annotation | Hs00985319_m1 |
| 62 | SAE1 | SUMO1 activating enzyme subunit 1, Gene hCG20373 Celera Annotation | Hs01062484_g1 |
| 63 | CLK4 | CDC-like kinase 4, Gene hCG20100 Celera Annotation | Hs00982806_m1 |
| 64 | STMN1 | stathmin 1, Gene hCG23745 Celera Annotation | Hs01027515_gH |
| 65 | DTYMK | deoxythymidylate kinase (thymidylate kinase), Gene hCG93868 Celera Annotation | Hs00992744_m1 |
| 66 | PRPF4 | PRP4 pre-mRNA processing factor 4 homolog (yeast), Gene hCG29193 Celera Annotation | HS00190796_m1 |
| 67 | TBC1D9B | TBC1 domain family, member 9B (with domain), Gene hCG15288 Celera Annotation | Hs00209268_m1 |
| 68 | FOXK2 | forkhead box K2, Gene hCG30380 Celera Annotation | Hs00895533_m1 |
| 69 | PAQR4 | progestin and adipoQ receptor family member IV,Gene hCG1778952 Celera Annotation | Hs00373823_m1 |
| 70 | POLE3 | polymerase (DNA directed), epsilon 3 (p17 subunit), Gene hCG29189 Celera Annotation | Hs00794385_m1 |
| 71 | CKS1A | CDC28 protein kinase regulatory subunit iB,Gene hCG24513 Celera Annotation, Gene hCG21562 Celera Annotation, Gene hCG1739274 Celera Annotation | Custom Assay |
| 72 | TEP1 | telomerase-associated protein 1, Gene hCG38226 Celera Annotation | Hs00200091_m1 |
| 73 | NDUFA1 | NADH dehydrogenase (ubiquinone) 1 alpha subcomplex, 1, 7.5kDa, Gene hCG23184 Celera Annotation | Hs00244980_m1 |
| 74 | RRM2 | ribonucleotide reductase M2, Gene hCG23833 Celera Annotation | Hs01072069_g1 |
| 75 | SERPINH1 | serpin peptidase inhibitor, clade H (heat shock protein 47), member 1, (collagen binding protein 1), Gene hCG26462 Celera Annotation | Hs00241844_m1 |
| 76 | STARD4 | StAR-related lipid transfer (START) domain containing 4Gene hCG37443 Celera Annotation | Hs00287823_m1 |
| 77 | LOC729678 | | Hs03678601_g1 |
| 78 | FBX045 | F-box protein 45, Gene hCG1734196 Celera Annotation | Hs00397889_m1 |
| 79 | TMEM201 | transmembrane protein 201, Gene hCG1748748 Celera Annotation | Hs00420510_m1 |
| 80 | ASFIB | ASF1 anti-silencing function 1 homolog B (S. cerevisiae), Gene hCG27531 Celera Annotation | Hs00216780_m1 |
| 81 | GMPS | guanine monphosphate synthetaseGene hCG1811302 Celera Annotation | Hs00269500_m1 |
| 82 | RCC2 | regulator of chromosome condensation 2, Gene hCG25129 Celera Annotation | Hs00603046_m1 |
| 83 | EBNA1BP2 | EBNA1 binding protein 2, Gene hCG2031782 Celera Annotation | Hs00199133_m1 |
| 84 | SDCCAG3 | serologically defined colon cancer antigen 3, Gene hCG2039971 Celera Annotation | Hs00981269_g1 |
| 85 | BID | BH3 interacting domain death agonist, Gene hCG21529 Celera Annotation | Hs00609692_m1 |
| 86 | GDPD2 | glycerophosphodiester phosphodiesterase domain containing 2, Gene hCG15349 Celera Annotation | Hs00214532_m1 |
| 87 | LRPPRC | leucine-rich PPR-motif containing,Gene hCG16810 Celera Annotation | Hs00370167_m1 |
| 88 | GTPBP4 | GTP binding protein 4, Gene hCG24698 Celera Annotation | Hs00202558_m1 |
| 89 | CCDC43 | coiled-coil domain containing 43, Gene hCG1771375 Celera Annotation | Hs00327475_m1 |
| 90 | MCM3 | minichromosome maintenance complex component 3, Gene hCG22498 Celera Annotation | Hs00172459_m1 |

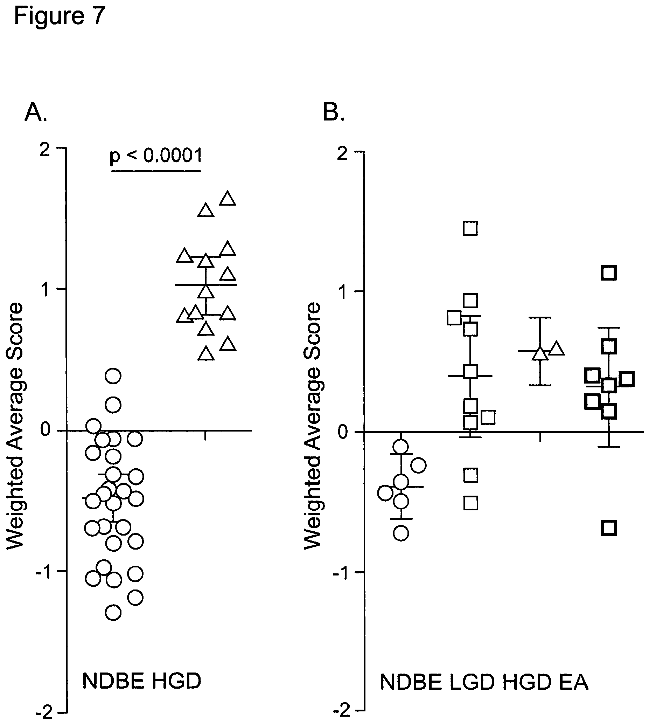

This gene expression signature separated the 13 HGD samples from 28 NDBE samples on the microarray gene expression dataset (p < 0.0001). This classifier gave no HGD misclassifications and only 2 NDBE misclassifications (Figure 7A). When the signature was applied to the remaining 26 samples within the microarray gene expression dataset, it classified the remaining 6 NDBE as 'low risk', 8 out of the 10 LGD as 'high risk', both the HGD as 'high risk' and 7 out of 8 EA as 'high risk' (Figure 7B).

### Pathway Analysis

Pathway analysis of all genes in the signature (GeneGo Software; http://www.genego.com) revealed that the RAN (RAs-regulated Nuclear protein) regulation pathway (p < 0.0001) was the most significantly enriched pathway within this 90-gene set. Other significantly enriched pathways included DNA damage, apoptosis and survival, and cell cycle transport pathways (Table B).

Interestingly, the oncogene, c-MYC, was found to regulate almost a third of the genes within the 90-gene set (Table B). Other potentially interesting transcription factors regulating genes in this signature included HNF4-alpha, SP1, NF-Y, E2F1, p53, ESR1 and HIF1A.

**Table B. Analysis of significantly-enriched pathways associated with the 90-gene signature and transcription factors involved in regulating genes in the 90-gene set.**

| **Pathway** | | **p value** |
|---|---|---|
| RAN regulation pathway | | 7.54x10⁻⁵ |
| NHEJ mechanisms of DSBs repair | | 8.93x10⁻⁵ |
| DNA damage induced responses | | 7.64x10⁻⁴ |
| TNFR1 signaling pathway | | 1.05x10⁻³ |
| Delta508-CFTR traffic | | 1.63x10⁻³ |
| Nucleocytoplasmic transport of CDK/Cyclins | | 1.90x10⁻³ |
| DNA damage induced apoptosis | | 3.52x10⁻³ |

| **Transcripetion Factor** | **No. of genes regulated** | **p value** |
|---|---|---|
| c-MYC | 29 | 5.16x10⁻⁸² |
| HNF4-alpha | 28 | 4.26x10⁻⁹ |
| SP1 | 21 | 7.20x10⁻⁵⁹ |
| NF-Y | 19 | 3.783x10⁻⁵⁸ |
| E2F1 | 15 | 8.72x10⁻⁴² |
| p53 | 13 | 3.81x10⁻³¹ |
| ESR1 | 13 | 3.81x10⁻³¹ |
| HIF1A | 12 | 2.46x10⁻³³ |

### Independent Validation

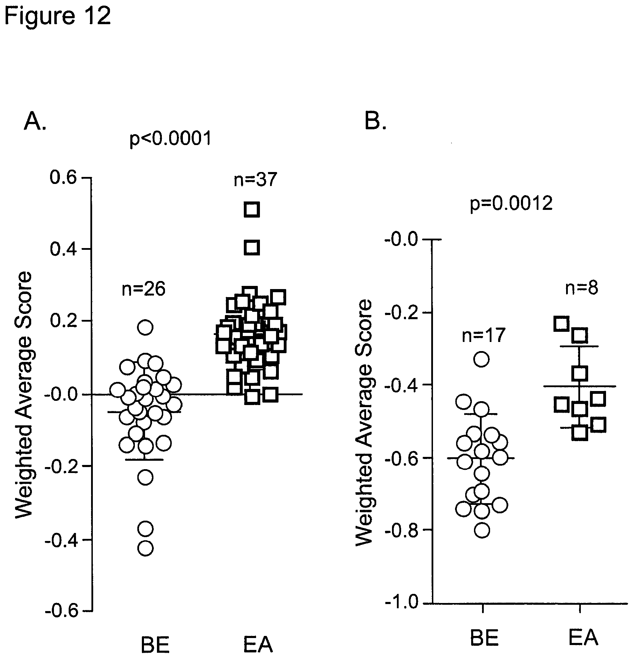

Analysis of published datasets revealed that the 90-gene signature was able to significantly separate NDBE samples from EA samples on 2 published datasets (p=0.0012) Figure 11 (Supplementary Figure 2)^{25, 26}. This was remarkably robust since not all 90 probes (genes) were present within the microarray probe-sets due to the different platforms used for these publically available datasets. In the Greenawalt *et al.* dataset Figure 12 (Supplementary Figure 3A) 55 out of 90 probes were present and the Wang *et al* dataset Figure 12 (Supplementary Figure 3B) contained 39 out of 90 probes. The lack of complete mapping of the probes may explain the overlap between groups.

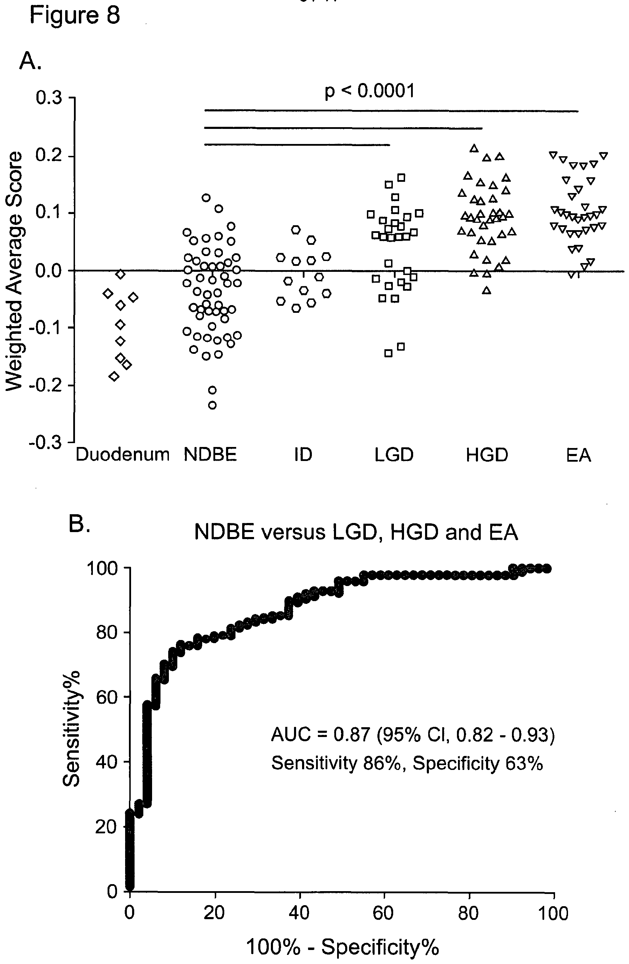

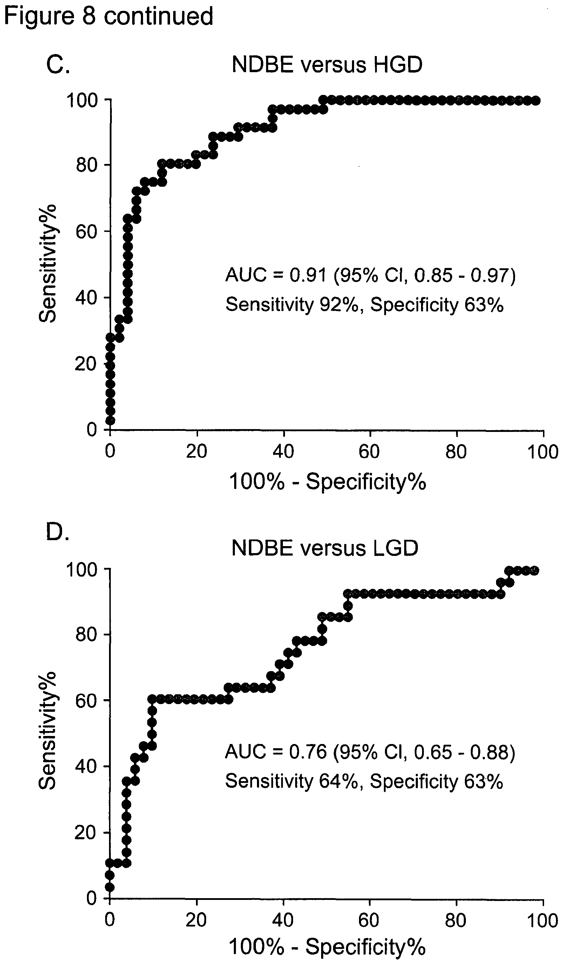

In a further independent set of 169 samples the 90-gene signature was able to successfully separate the samples based on the weighted average score defined as 'high risk' (above the line) and 'low risk' (below the line) Figure 8 (Figure 3A). All the duodenal samples, which are considered as normal columnar control tissues when compared to BE samples, were scored as 'low risk'. The 90-gene signature separated BE with no dysplasia samples from BE with dysplasia and cancer samples with an area under the curve of 0.87 (95% CI, 0.82-0.93; sensitivity of 86% and specificity of 63%), Figure 8 (Figure 3B).

The 90-gene signature was then assessed separately for LGD and HGD. The 90-gene signature successfully separated BE with no dysplasia from HGD samples with an area under the curve of 0.91 (95% CI, 0.85-0.97; sensitivity of 92%, specificity of 63%) Figure 8 (Figure 3C). For LGD samples, which (as discussed earlier) are more difficult to categorize based on histology, there was an area under the curve of 0.76 (95% CI, 0.65-0.88; sensitivity of 64% and specificity of 63%), Figure 8 (Figure 3D). For these LGD samples, 18 of the 28 (64%) were classified as 'high risk' and 5 (18%) as 'low risk'. Follow up data was available for 42 of the NDBE and LGD patients. For 22 of the individuals whose samples were classified as 'high risk' and for whom we have follow up data, 5 (23%) progressed to HGD or EA compared to only 1 (5%) out of the 20 individuals that were classified as 'low risk' using the 90-gene signature (p=0.0244), Figure 13 (Supplementary Figure 4). Furthermore, in 13 cases in which the pathologist was unable to make a definite diagnosis of dysplasia (indefinite for dysplasia), the gene signature classified 6 of these as 'high risk' samples - on follow up, 4 out of these 6 patients had LGD on repeat biopsy samples within a 12 month period. On the other hand, 7 indefinite for dysplasia cases were classified as 'low risk' by the signature. During the 12 month surveillance follow up period of these cases, 6 were downgraded to non-dysplastic and one patient was identified as LGD.

### Discussion

This example has developed and validated a class prediction model for BE using gene expression microarray data. The aim was for this gene expression signature to identify dysplastic samples in BE and in particular to distinguish the 'true' or highest risk LGD samples. These LGD in the 'high risk' gene signature category should be analogous to the 15% given a consensus diagnosis in the Curvers study with a 13.4% annual risk of progression, 6 and thereby provide a more objective biomarker for risk stratification.

It is known that gene signatures identify sub-types of disease and can be useful in distinguishing benign from malignant conditions. However use of a gene classifier in a pre-malignant condition to assess risk of progression to cancer has not yet been described in the literature. Strengths of this study include the use of a robust bio-informatic approach to identify this gene signature. A 90 gene signature was selected based on a robust statistical analysis which demonstrated an improved performance compared to shorter signatures; however some degree of over-fitting is seen and the net increase in AUC for signatures above 60 genes is small Figures 10 and 11 (supplementary Figures 1 and 2). Care was also taken to ensure that each sample in the training and test set was verified by expert gastrointestinal pathologists prior to mRNA extraction. A high standard for selection of samples used for the training set is crucial for generating a clinically relevant biomarker. Whilst it is advantageous for a gene signature to work well using a simple weighted-average of values, testing different classification methods on the gene expression microarray training set suggested slightly better results could be obtained with more sophisticated classifier methods. Therefore, further improvement in performance might be possible if a microfluidic chip dataset was used to train an SVM, or other classifier, specifically for the 90-gene signature on the microfluidic platform.

The weaknesses in this study are inherent to microarray based gene classifiers and include complex analysis in the interpretation of the data, the requirement of fresh frozen samples which can be difficult to obtain in some centres that lack liquid nitrogen or adequate storage facilities for these samples. However, fresh frozen samples are increasingly being used clinically in view of the explosion of molecular diagnostic tests and there are methods of preserving RNA without the need for immediate flash freezing, for example using the preservative, RNA later. The samples in both the training and validation set were enriched for dysplasia when compared to the general population with Barrett's esophagus, but this was done as this is the initial study. It would therefore be important to validate this classifier in further prospective studies. Pathway analysis of the genes on the signature highlighted possible novel pathways in the pathogenesis of EA including the RAN regulation pathway. RAN is a small GTP-binding protein that is involved in the nuclear transport of proteins by interacting with karyopherins^{27, 28}. The analysis of transcription factors associated with the genes in the 90-gene set has shown c-MYC as the most significant transcription factor regulating 29 of the genes on the signature. Dysregulation of c-MYC has been implicated in Barrett's esophagus carcinogenesis^{29, 30} and its role as a biomarker in Barrett's esophagus has been proposed both as an immunohistochemical marker^{29, 31} and as a fluorescence in situ hybridization probe to detect HGD³²⁻³⁴. No definite conclusions have been yet been made but previous studies along with this study highlight the importance of revisiting the role of c-MYC in Barrett's carcinogenesis.

With regards to clinical utility, this 90-gene signature for assessing the degree of molecular abnormality would be most useful when there is a diagnosis of LGD, as illustrated in the proposed clinical pathway (Figure 9). The results also suggest that the gene signature may be a useful adjunct in cases of indefinite for dysplasia. When the diagnosis of NDBE or HGD is made, the management is more clear-cut and we are not proposing use of the gene classifier for these patients. In the case of LGD and indefinite for dysplasia, the pathologist may have difficulty in assigning the diagnostic category and the managing clinician is usually left in a dilemma. In the US, clinicians are starting to treat patients with LGD with radiofrequency ablation therapy (RFA) 35 however whether this practice is advisable for LGD is questionable given the variability in diagnosis and the requirement for long term follow-up which starts to alter the cost-economics. In Europe and the United Kingdom, RFA is generally reserved for HGD 36 but with data showing that within the LGD category there are individuals at significant risk⁶ it would be valuable to identify these particular individuals. Hence, our 90-gene signature could enable the clinician to focus on the patients with LGD with a gene signature resembling HGD and hence advise endoscopic therapy or close surveillance. For those with LGD and a 'lowrisk' gene signature we would suggest continued surveillance, in keeping with current clinical practice, and avoid unnecessary invasive endoscopic procedures in this sub-group.

It also worth pointing out that there are cases in the non-dysplastic group that appear to be at 'high risk' according to our signature presumably because the gene expression changes predate the cytomorphological features of dysplasia. It is therefore possible that the use of a signature like this could help to re-think the classification systems for risk stratification and move away from a histopathologial grading with all the drawbacks that entails.

### References

1. Hvid-Jensen F, Pedersen L, Drewes AM, et al. Incidence of adenocarcinoma among patients with Barrett's esophagus. N Engl J Med 2011;365:1375-83.
2. Yousef F, Cardwell C, Cantwell MM, et al. The incidence of esophageal cancer and high-grade dysplasia in Barrett's esophagus: a systematic review and meta-analysis. Am J Epidemiol 2008;168:237-49.
3. Bhat S, Coleman HG, Yousef F, et al. Risk of malignant progression in Barrett's esophagus patients: results from a large population-based study. J Natl Cancer Inst 2011;103:1049-57.
4. Desai TK, Krishnan K, Samala N, et al. The incidence of oesophageal adenocarcinoma in non-dysplastic Barrett's oesophagus: a meta-analysis. Gut 2012;61:970-6.
5. Schlemper RJ, Riddell RH, Kato Y, et al. The Vienna classification of gastrointestinal epithelial neoplasia. Gut 2000;47:251-5.
6. Curvers WL, ten Kate FJ, Krishnadath KK, et al. Low-grade dysplasia in Barrett's esophagus: overdiagnosed and underestimated. Am J Gastroenterol 2010;105:1523-30.
7. Wani S, Falk GW, Post J, et al. Risk factors for progression of low-grade dysplasia in patients with Barrett's esophagus. Gastroenterology 2011;141:1179-86, 1186.e1.
8. Kerkhof M, van Dekken H, Steyerberg EW, et al. Grading of dysplasia in Barrett's oesophagus: substantial interobserver variation between general and gastrointestinal pathologists. Histopathology 2007;50:920-7.
9. Skacel M, Petras RE, Gramlich TL, et al. The diagnosis of low-grade dysplasia in Barrett's esophagus and its implications for disease progression. Am J Gastroenterol 2000;95:3383-7.
10. Montgomery E, Bronner MP, Goldblum JR, et al. Reproducibility of the diagnosis of dysplasia in Barrett esophagus: a reaffirmation. Hum Pathol 2001;32:368-78.
11. Reid BJ, Haggitt RC, Rubin CE, et al. Observer variation in the diagnosis of dysplasia in Barrett's esophagus. Hum Pathol 1988;19:166-78.
12. Sikkema M, Looman CW, Steyerberg EW, et al. Predictors for neoplastic progression in patients with Barrett's Esophagus: a prospective cohort study. Am J Gastroenterol 2011;106:1231-8.
13. Phoa K, van Vilsteren F, Pouw R, et al. Radiofrequency Ablation in Barrett's Esophagus With Confirmed Low-Grade Dysplasia: Interim Results of a European Multicenter Randomized Controlled Trial (SURF). Digestive Diseases Week 2013, 2013.
14. Hur C, Choi SE, Rubenstein JH, et al. The cost effectiveness of radiofrequency ablation for Barrett's esophagus. Gastroenterology 2012;143:567-75.
15. Bergman JJ, Corley DA. Barrett's esophagus: who should receive ablation and how can we get the best results? Gastroenterology 2012;143:524-6.
16. Sørlie T, Perou CM, Tibshirani R, et al. Gene expression patterns of breast carcinomas distinguish tumor subclasses with clinical implications. Proc Natl Acad Sci U S A 2001;98:10869-74.
17. van't Veer U, Dai H, van de Vijver MJ, et al. Gene expression profiling predicts clinical outcome of breast cancer. Nature 2002;415:530-6.
18. Beer DG, Kardia SL, Huang CC, et al. Gene-expression profiles predict survival of patients with lung adenocarcinoma. Nat Med 2002;8:816-24.
19. Salazar R, Roepman P, Capella G, et al. Gene expression signature to improve prognosis prediction of stage II and III colorectal cancer. J Clin Oncol 2011;29:17-24.
20. van de Vijver MJ, He YD, van't Veer LJ, et al. A gene-expression signature as a predictor of survival in breast cancer. N Engl J Med 2002;347:1999-2009.
21. Alexander EK, Kennedy GC, Baloch ZW, et al. Preoperative diagnosis of benign thyroid nodules with indeterminate cytology. N Engl J Med 2012;367:705-15.
22. R: A Language and Environment for Statistical Computing, R Foundation for Statistical Computing, 2013. Volume 2013, 2013.
23. Smyth GK. Linear models and empirical bayes methods for assessing differential expression in microarray experiments. Stat Appl Genet Mol Biol 2004;3:Article3.
24. Spurgeon SL, Jones RC, Ramakrishnan R. High throughput gene expression measurement with real time PCR in a microfluidic dynamic array. PLoS One 2008;3:e1662.
25. Greenawalt DM, Duong C, Smyth GK, et al. Gene expression profiling of esophageal cancer: comparative analysis of Barrett's esophagus, adenocarcinoma, and squamous cell carcinoma. Int J Cancer 2007;120:1914-21.
26. Wang S, Zhan M, Yin J, et al. Transcriptional profiling suggests that Barrett's metaplasia is an early intermediate stage in esophageal adenocarcinogenesis. Oncogene 2006;25:3346-3356.
27. Moore MS, Blobel G. A G protein involved in nucleocytoplasmic transport: the role of Ran. Trends Biochem Sci 1994;19:211-6.
28. Shamsher MK, Ploski J, Radu A. Karyopherin beta 2B participates in mRNA export from the nucleus. Proc Natl Acad Sci U S A 2002;99:14195-9.
29. Tselepis C, Morris CD, Wakelin D, et al. Upregulation of the oncogene c-myc in Barrett's adenocarcinoma: induction of c-myc by acidified bile acid in vitro. Gut 2003;52:174-80.
30. Boult JK, Tanière P, Hallissey MT, et al. Oesophageal adenocarcinoma is associated with a deregulation in the MYC/MAX/MAD network. Br J Cancer 2008;98:1985-92.
31. Schmidt MK, Meurer L, Volkweis BS, et al. c-Myc overexpression is strongly associated with metaplasia-dysplasia-adenocarcinoma sequence in the esophagus. Dis Esophagus 2007;20:212-6.
32. Brankley SM, Fritcher EG, Smyrk TC, et al. Fluorescence in situ hybridization mapping of esophagectomy specimens from patients with Barrett's esophagus with high-grade dysplasia or adenocarcinoma. Hum Pathol 2012;43:172-9.
33. Rygiel AM, Milano F, Ten Kate FJ, et al. Assessment of chromosomal gains as compared to DNA content changes is more useful to detect dysplasia in Barrett's esophagus brush cytology specimens. Genes Chromosomes Cancer 2008;47:396-404.
34. Rygiel AM, Milano F, Ten Kate FJ, et al. Gains and amplifications of c-myc, EGFR, and 20.q13 loci in the no dysplasia-dysplasia-adenocarcinoma sequence of Barrett's esophagus. Cancer Epidemiol Biomarkers Prev 2008;17:1380-5.
35. Bulsiewicz W, Pasricha S, Komanduri S, et al. Durability of Reversion to Squamous Mucosa After Successful Eradication of Barrett's Esophagus (BE) With Radiofrequency Ablation (RFA): Results from the U.S. RFA Registry. Digestive Diseases Week 2013, 2013.
36. Haidry RJ, Dunn JM, Butt MA, et al. Radiofrequency Ablation and Endoscopic Mucosal Resection for Dysplastic Barrett's Esophagus and Early Esophageal Adenocarcinoma: Outcomes of the UK National Halo RFA Registry. Gastroenterology 2013;145:87-95.

## Claims

1. A method of aiding the diagnosis of dysplasia in a subject, said method comprising:
(a) providing an *in vitro* sample previously collected from the oesophagus of said subject;
(b) assaying said sample for expression of each of the genes shown in the '40 genes' column of Table 1;
(c) normalising the expression levels of the genes in part (b) to expression levels of reference gene(s) from a non-dysplastic sample;
(d) determining from the normalised expression levels of (c) a gene signature score for the sample, wherein a gene signature score greater than a reference threshold indicates presence of dysplasia in said subject.

2. A method according to claim 1 wherein said *in vitro* sample is a biopsy.

3. A method according to claim 2 wherein said biopsy is a pinch biopsy, or an endoscopic brushing.

4. A method according to any of claims 1 to 3 wherein assaying for expression of said genes is carried out by quantification of nucleic acid such as RNA in said *in vitro* sample.

5. A method according to any of claims 1 to 4 wherein assaying for expression of said genes is carried out using qPCR analysis.

6. A method according to any preceding claim wherein said assay includes Gaussian normalisation.

7. A method according to any preceding claim wherein assaying for expression of said genes is carried out using an expression array.

8. A method according to any preceding claim wherein assaying for expression of said genes is carried out using RNA sequencing such as RNASeq.

9. A method according to any preceding claim wherein the expression of the genes is assayed by detection of the probe(s) for said genes as shown in Table 2.

10. A method according to any preceding claim wherein said *in vitro* sample comprises RNA extracted from cell(s) of said subject.

11. A method according to any preceding claim wherein said subject has Barrett's Oesophagus.

12. An array consisting of nucleic acid probe(s) capable of detecting RNA from each of the genes shown in the '40 genes' column of Table 1, wherein said array comprises a biochip to which the nucleic acid probe(s) are immobilised.

13. A method according to any of claims 1 to 11 wherein step (b) comprises contacting nucleic acid of said sample with one or more isolated probe(s) to allow hybridisation/ binding to said probe(s), and then reading out said binding/hybridisation.

14. A method of aiding identification of a subject at risk of developing oesophageal adenocarcinoma, said method comprising performing the method according to any of claims 1 to 11 wherein presence of dysplasia in said subject indicates that said subject is at risk of developing oesophageal adenocarcinoma.

15. A computer program product operable, when executed on a computer, to perform the method steps (c) to (d) of any of the claims 1 to 11.

## Patentansprüche

1. Verfahren zum Unterstützen der Dysplasie-Diagnose bei einem Subjekt, wobei das Verfahren Folgendes umfasst:
a) Bereitstellen einer *In-vitro-Probe,* die zuvor aus dem Ösophagus des Subjekts entnommen wurde;
b) Testen der Probe auf eine Expression jedes der in der "40-Gene"-Spalte von Tabelle 1 dargestellten Gene;
c) Normalisieren der Expressionsspiegel der Gene in Teil b) auf die Expressionsspiegel von (einem) Referenzgen(en) aus einer nicht dysplastischen Probe;
d) Bestimmen, aus den normalisierten Expressionsspiegeln von c), eines Gensignatur-Scores für die Probe, wobei ein Gensignatur-Score von mehr als dem Referenzschwellenwert das Vorhandensein von Dysplasie bei dem Subjekt anzeigt.

2. Verfahren nach Anspruch 1, wobei die *In-vitro-Probe* eine Biopsie ist.

3. Verfahren nach Anspruch 2, wobei die Biopsie eine Stanzbiopsie oder eine endoskopische Bürstenbiopsie ist.

4. Verfahren nach einem der Ansprüche 1 bis 3, wobei das Testen auf eine Expression der Gene durch Quantifizierung von Nukleinsäure wie RNA in der *In-vitro-Probe* durchgeführt wird.

5. Verfahren nach einem der Ansprüche 1 bis 4, wobei das Testen auf eine Expression der Gene unter Verwendung einer qPCR-Analyse durchgeführt wird.

6. Verfahren nach einem der vorstehenden Ansprüche, wobei der Test eine Gaußsche Normalisierung beinhaltet.

7. Verfahren nach einem der vorstehenden Ansprüche, wobei das Testen auf eine Expression der Gene unter Verwendung eines Expressions-Arrays durchgeführt wird.

8. Verfahren nach einem der vorstehenden Ansprüche, wobei das Testen auf eine Expression der Gene unter Verwendung von RNA-Sequenzierung wie beispielsweise RNASeq durchgeführt wird.

9. Verfahren nach einem der vorstehenden Ansprüche, wobei die Expression der Gene durch Nachweis der Sonde(n) für die Gene wie in Tabelle 2 dargestellt getestet wird.

10. Verfahren nach einem der vorstehenden Ansprüche, wobei die *In-vitro-Probe* aus (einer) Zelle(n) des Subjekts extrahierte RNA umfasst.

11. Verfahren nach einem der vorstehenden Ansprüche, wobei das Subjekt Barrett-Ösophagus hat.

12. Array, bestehend aus (einer) Nukleinsäuresonde(n), das RNA von jedem der in der "40-Gene"-Spalte von Tabelle 1 dargestellten Gene nachweisen kann, wobei das Array einen Biochip umfasst, an dem die Nukleinsäuresonde(n) immobilisiert ist/sind.

13. Verfahren nach einem der Ansprüche 1 bis 11, wobei Schritt b) das In-Kontakt-Bringen von Nukleinsäure der Probe mit einer oder mehreren isolierten Sonde(n) umfasst, um eine Hybridisierung/Bindung an die Sonde(n) zu erlauben, sowie das anschließende Auslesen der Bindung/Hybridisierung.

14. Verfahren zum Unterstützen der Identifikation eines Subjekts, bei dem das Risiko der Entwicklung eines ösophagealen Adenokarzinoms besteht, wobei das Verfahren das Durchführen des Verfahrens nach einem der Ansprüche 1 bis 11 umfasst, wobei das Vorhandensein von Dysplasie bei dem Subjekt anzeigt, dass bei dem Subjekt das Risiko besteht, ein ösophageales Adenokarzinom zu entwickeln.

15. Computerprogrammprodukt, dass bei Ausführung auf einem Computer funktionsbereit ist, um die Verfahrensschritte c) bis d) nach einem der Ansprüche 1 bis 11 durchzuführen.

## Revendications

1. Procédé d'aide au diagnostic de dysplasie chez un sujet, ledit procédé comprenant :
(a) la fourniture d'un échantillon *in vitro* prélevé au préalable à partir de l'œsophage dudit sujet ;
(b) le dosage dudit échantillon à la recherche d'une expression de chacun des gènes présentés dans la colonne « 40 gènes » du tableau 1 ;
(c) la normalisation des niveaux d'expression des gènes dans la partie (b) pour des niveaux d'expression de gène(s) de référence à partir d'un échantillon non dysplasique ;
(d) la détermination, à partir des niveaux d'échantillons normalisés de (c), d'un score de signature de gène pour l'échantillon, dans lequel un score de signature de gène supérieur à un seuil de référence indique la présence d'une dysplasie chez ledit sujet.

2. Procédé selon la revendication 1, dans lequel ledit échantillon *in vitro* est une biopsie.

3. Procédé selon la revendication 2, dans lequel ladite biopsie est une biopsie à la pince, ou un brossage endoscopique.

4. Procédé selon l'une quelconque des revendications 1 à 3, dans lequel le dosage à la recherche de l'expression desdits gènes est réalisé par quantification d'acide nucléique tel que l'ARN dans ledit échantillon *in vitro.*

5. Procédé selon l'une quelconque des revendications 1 à 4, dans lequel le dosage à la recherche de l'expression desdits gènes est réalisé à l'aide d'une analyse qPCR.

6. Procédé selon une quelconque revendication précédente, dans lequel ledit dosage inclut une normalisation gaussienne.

7. Procédé selon une quelconque revendication précédente, dans lequel le dosage à la recherche de l'expression desdits gènes est réalisé à l'aide d'un réseau d'expression.

8. Procédé selon une quelconque revendication précédente, dans lequel le dosage à la recherche de l'expression desdits gènes est réalisé à l'aide d'un séquençage d'ARN tel que le RNAseq.

9. Procédé selon une quelconque revendication précédente, dans lequel l'expression des gènes est dosée par détection de sonde(s) pour lesdits gènes tels que présenté dans le tableau 2.

10. Procédé selon une quelconque revendication précédente, dans lequel ledit échantillon *in vitro* comprend de l'ARN extrait de cellule(s) dudit sujet.

11. Procédé selon une quelconque revendication précédente, dans lequel ledit sujet est atteint d'un œsophage de Barrett.

12. Réseau constitué de sonde(s) nucléique(s) apte à détecter de l'ARN provenant de chacun des gènes présentés dans la colonne « 40 gènes » du tableau 1, dans lequel ledit réseau comprend une puce à ADN sur laquelle la ou les sondes nucléiques sont immobilisées.

13. Procédé selon l'une quelconque des revendications 1 à 11, dans lequel l'étape (b) comprend la mise en contact d'acide nucléique dudit échantillon avec une ou plusieurs sondes isolées pour permettre l'hybridation/la liaison à ladite ou auxdites sondes, puis la lecture de ladite liaison/hybridation.

14. Procédé d'aide à l'identification d'un sujet à risque de développer un adénocarcinome de l'œsophage, ledit procédé comprenant la mise en œuvre du procédé selon l'une quelconque des revendications 1 à 11 dans lequel la présence d'une dysplasie chez ledit sujet indique que ledit sujet est à risque de développer un adénocarcinome de l'œsophage.

15. Produit de programme informatique pouvant fonctionner, lorsqu'il est exécuté sur un ordinateur, pour mettre en œuvre les étapes (c) à (d) du procédé selon l'une quelconque des revendications 1 à 11.
